(19) Europäisches Patentamt
European Patent Office
Office européen des brevets

(11) EP 4 255 626 B1

(12) **EUROPEAN PATENT SPECIFICATION**

(45) Date of publication and mention of the grant of the patent:
**12.02.2025 Bulletin 2025/07**

(21) Application number: **21816114.9**

(22) Date of filing: **03.12.2021**

(51) International Patent Classification (IPC):
***B01J 35/00*** (2024.01) ***B01J 37/02*** (2006.01)

(52) Cooperative Patent Classification (CPC):
**B01J 35/39; B01J 37/0215**

(86) International application number:
**PCT/EP2021/084214**

(87) International publication number:
**WO 2022/122590 (16.06.2022 Gazette 2022/24)**

(54) **HETEROGENEOUS REDOX CATALYTIC SYSTEM**

KATALYTISCHE SYSTEME AUF DER BASIS VON ISOALLOXINEN UND DEREN VERWENDUNG

SYSTÈMES CATALYTIQUES À BASE D'ISOALLOXAZINE ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2020 EP 20383067**

(43) Date of publication of application:
**11.10.2023 Bulletin 2023/41**

(73) Proprietors:
- **Asociación Centro de Investigación Cooperativa en Biomateriales - CIC biomaGUNE
20014 San Sebastián (ES)**
- **Fundacion Donostia International Physics Center -
DIPC
20018 Donostia-San Sebastián (ES)**
- **Universidad del País Vasco/Euskal Herriko Unibertsitatea
48940 Leoia, Bikaia (ES)**

(72) Inventors:
- **LÓPEZ-GALLEGO, Fernando
20014 DONOSTIA-SAN SEBASTIÁN (ES)**
- **SALASSA, Luca
20018 DONOSTIA-SAN SEBASTIÁN (ES)**

(74) Representative: **ZBM Patents - Zea, Barlocci & Markvardsen
Rambla de Catalunya, 123
08008 Barcelona (ES)**

(56) References cited:
- **ALONSO-DE?CASTRO SILVIA ET AL: "Bioorthogonal Catalytic Activation of Platinum and Ruthenium Anticancer Complexes by FAD and Flavoproteins", ANGEWANDTE CHEMIE INTERNATIONAL EDITION, vol. 57, no. 12, 12 March 2018 (2018-03-12), DE, pages 3143 - 3147, XP055803929, ISSN: 1433-7851, DOI: 10.1002/anie.201800288**
- **BENÍTEZ-MATEOS ET AL: "Selective Immobilization of Fluorescent Proteins for the Fabrication of Photoactive Materials", MOLECULES, vol. 24, no. 15, 1 August 2019 (2019-08-01), DE, pages 2775, XP055803928, ISSN: 1433-1373, DOI: 10.3390/molecules24152775**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field

The present invention relates to catalysis field. In particular, the present invention refers to isoalloxazine-based catalytic systems, to processes for their preparation as well as to their use in therapy, diagnostic and REDOX industrial processes.

### Background Art

Isoalloxazines (so-called "flavins") are yellow chromophores derived from riboflavin (vitamin B2). The tricyclic isoalloxazine ring of formula (I) is the catalytic core of these molecules:

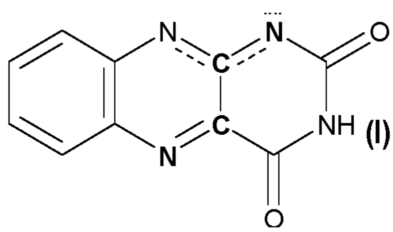

(I)

Its N5 and N1 atoms (bold-highlighted) represent the catalytically reactive positions, because they constitute the conjugated p electron system that can exist in oxidized, one-electron reduced, and two electron reduced forms. The double asterisk at the N1 atom indicates that, when the double bond is located between N10 and C10a, then it has to be bound to a hydrogen atom. The single asterisk at the N10 atom indicates that, when the double bond is located between N1 and C10a, then N10 has to bind to a substituent which takes a particular meaning depending on the particular isoalloxazine. The remaining atoms forming the core can also be substituted, by different groups, depending on the particular isoalloxazine.

Isolloxazine-based catalysts are defined as requiring the isoalloxazine (I) as prosthetic group for their activity. Indeed, for many, if not most isolloxazine-based catalytic systems, the catalytic mechanism entails the oxidation of the substrate with formation of the reaction product, which may be released immediately or remain bound to the active site until the reaction cycle is completed. Then, the reduced isoalloxazine reacts with an electron-accepting substrate (e.g., oxygen), regenerating its oxidized state. All the reaction types that reduce isoalloxazines can, in principle, also go in reverse.

Isoalloxazines are involved in a broad range of physiological activities, including energy, secondary and xenobiotic metabolisms, biosynthesis of metabolites and antibiotics, and in signaling and photoreceptor activities. Knowledge of isoalloxazine-dependent catalysts can be applied in a wide range of applications, such as drug discovery and synthesis, industrial biocatalysis, bioenergy, and biosensing tools.

For example, attention has been paid in the last years on the REDOX activity of some isoalloxazine-based catalysts towards Pt(IV) prodrugs, in an attempt to avoid the direct administration of Pt(II), widely reported as highly toxic. Thus, for instance, the ability of riboflavin for catalysing the reduction of a Pt(IV) prodrug to Pt(II) in an *in vitro* test has already been reported by Alonso-de Castro S. and colleagues (cf. Alonso-de Castro S. et al., 2108), wherein cells were incubated with Pt(IV) prodrug and the riboflavin. No formulation was made in this report to avoid the systemic administration of both the Pt(IV) prodrug and the catalyst.

In spite of the *in vitro* catalytic activity exhibited by these catalysts, however, their use in industrial, diagnostics or therapeutic applications is very limited.

On one hand, the isoalloxazine-based catalysts have to be stable and efficient enough under reaction conditions which can be extremely negative for the catalytic activity. On the other hand, this stability and efficiency do not have to hinder the accessibility of the catalyst to the substrate, because the catalytic yield can be negatively affected.

In addition to the above, the catalysts have to be easily removable from the reaction medium and the amount of the catalyst along the process has to be guaranteed to avoid a reduction in the catalytic yield. And, in the particular case of their possible use in therapy, such as their use in reducing Pt(IV) prodrugs, further drawbacks are the impossibility of "controlling" both the catalyst concentration required to efficiently act and the place wherein the REDOX reaction has to take place when both, the catalyst and the substrate, are administered to a patient.

Therefore, there is still the need of catalytic systems which solve one or more of the above drawbacks related to their use in the industry and therapy.

**Summary of Invention**

The present inventors have found that when an isoalloxazine-based catalyst is reversibly immobilized by non-covalent interactions within a functionalized porous polymeric matrix: (a) the catalyst is stable within the matrix and exhibits a high catalytic efficiency; (b) the substrate can efficiently diffuse, through the pores, to be also immobilized and subjected to REDOX; and (c) the product resulting from the REDOX reaction can easily diffuse out from the porous polymeric matrix to the medium.

The present inventors have found that the polymeric matrix plays an essential role in the surprising catalytic behavior reported herein due to the functionalization with appropriate anchoring groups (required for the non-covalent reversible interaction with the catalyst) and electronic groups (required for the catalytic reaction), whose properties are complementary to those of the catalyst to be included.

In connection with the above, the present inventors found that there was a change in the morphology of the functionalized porous matrix (in the examples provided below with the positively charged amino groups, particularly diethylamineethyl, "DEAE" whose role was both anchoring and electron accepting), when the REDOX occurred, as shown in Fig.1, changes were not detected in dark. This was conclusive that the porous polymeric matrix had, in fact, a dual role: as chassis to support the catalytic reaction, but also as a source for accepting electrons, being the latter of special relevance in the catalytic activity of the system of the invention (without this source no possible REDOX would occur in the system). Therefore, the porous polymeric matrix, thanks to the inclusion of appropriate functional groups, at an appropriate amount, promotes the non-covalent reversible interaction but also provides the appropriate electronic environment, thus becoming an excellent scaffold for optimizing the activity of the catalyst.

Continuing with the polymeric matrix, its porous nature guarantees (a) the appropriate accessibility of the substrate to the catalyst, and (b) the easy diffusion of the product resulting from REDOX reactions to the bulk. Regarding (a), the substrate can easily diffuse through the pores into the matrix and to the vicinity of the catalyst, which is rapidly moving within the polymeric matrix due to the reversible interactions that bind the catalyst to the matrix, and can find the catalyst to be subjected to the intended REDOX reaction.

Altogether the catalytic system of the invention has important advantages in industry, because the catalyst activity can be appropriately controlled in space and time to operate, working for long periods of time, protected by the porous matrix, and efficiently converting the substrate thanks to the nature of the interaction as well as by the electronic source incorporated in the polymeric matrix.

Thus, in a first aspect the present invention provides a catalytic system comprising a REDOX catalyst and a porous polymeric matrix wherein

- the porous polymeric matrix has pores with size from 1 and 1000 nm and it is covalently functionalized with functional groups $R_1$ or, alternatively, with functional groups $R'_1$ and $R_2$;
- the REDOX catalyst comprises an isoalloxazine moiety which has one or more functional groups $R_3$;
- $R_1$ and $R_2$ are capable of forming non-covalent reversible interactions, selected from the group consisting of: electrostatic, hydrophobic and hydrogen bonds, with $R_3$;
- $R_1$ and $R_2$ functional groups are in a molar concentration excess with respect to the molar concentration of the REDOX catalyst;
- both $R_1$ and $R'_1$ are electron donor or electron acceptor groups having a REDOX potential between +2.70 to -2.5 V as determined by cyclic voltammetry using an Ag/AgCl electrode as reference.

The cyclic voltammetry measurements are performed at room temperature in DMSO solutions containing $[N^nBu_4][CF_3SO_3]$ (0.1 M) as the supporting electrolyte.

Cyclic voltammetry is performed in a three-electrode cell with a Teflon encapsulated carbon-glassy working electrode, a platinum-gauze counter electrode and a platinum quasi reference electrode. Prior to measurements, the glassy-carbon working electrode is polished according to the following procedure: manual rubbing with 0.3 $\mu$m $Al_2O_3$ slurry in water (eDAQ) for 2 min, then sonication in ultrapure water for 10 min, manual rubbing with 0.05 $\mu$m $Al_2O_3$ slurry in water (eDAQ) for 2 min, then sonication in ultrapure water for 10 min. The DMSO solution of the supporting electrolyte is introduced into the cell, and deaerated by bubbling Ar. The potential of the working electrode is cycled several times between the cathodic and anodic limits to determine the solvent window. The analyte formulated in a DMSO solution (in this case a monomer of the polymeric matrix functionalized with $R_1$ or $R'_1$) is then introduced into the cell at a concentration of about $1\times10^{-3}$ M and the voltammogram is recorded (0.1 V $s^{-1}$); then about$1\times10^{-3}$ of $Cp_2Fe$ (ferrocene) is added and a further voltammogram is recorded. Potentials are determined by placing the $Cp_2Fe^+/Cp_2Fe$ couple at 0.0 V. Under these experimental conditions, the one-electron oxidation of $Cp_2Fe$ occurs at E° = +0.50 V vs. Ag/AgCl. Assignment of $R_1$ and $R'_1$ REDOX peak is achieved by comparison with a solution with the monomer alone, under the same conditions.

Surprisingly, and contrary to what the skilled person in the art would expect, the present inventors have found that reversible non-covalent interactions, which are recognized as "weak interactions" in terms of energy (about 1-5 kcal/mol),

are strong enough to maintain the catalyst within the porous matrix and weak enough to allow short range movements of the catalyst, which has a direct positive impact on the catalytic conversion efficiency.

The present invention also provides in a second aspect a process for preparing a catalytic system as defined in the first aspect, the process comprising the mixing of the porous polymeric matrix and the catalyst, as defined in the first aspect of the invention, wherein the catalyst is added at a molar concentration in defect with respect to the molar concentration of "$R_1$" functional groups, or alternatively, with respect to $R'_1$ and $R_2$ functional groups, under appropriate conditions to allow the formation of the non-covalent reversible interactions between the catalyst and the functional groups of the polymeric matrix.

Herein, it is reported a new drug release approach for Pt anticancer agents that exploits unconventional catalytic reactions towards metal substrates confined into hydrogels. These uncommon reactions use metal complexes as substrates (rather than catalysts) and occur with high efficiency and selectivity even in biological environments. Mechanistic studies indicate that the catalytic active species in these reactions is the doubly reduced flavin form whose generation is dramatically accelerated by photoirradiation in the presence of electron donors.

So far, catalysis has been employed in drug release to accelerate the degradation of hydrogels and thus control the delivery of active agents. The example below show the catalytically-driven activation of a Pt(IV) prodrug complex (i.e., cis,cis,trans-$[Pt(NH_3)_2(Cl_2)(O_2CCH_2CH_2CO_2H)_2]$ where - $O_2CCH_2CH_2CO_2H$ = succinate) and subsequent release of cisplatin in a stimuli responsive manner, both by light and chemical activation (co-factor), using a catalytic system of the invention.

In the field of therapy, in addition to the advantages mentioned above, the catalytic system is also of great importance because together with the oxidizable or reducible prodrug, can be formulated in a safe porous polymeric matrix (applied to a medical device, for instance), and the complete conversion of the prodrug to the drug can be achieved by applying a low intensity radiation for a very short period of time, the drug diffusing through the pores and providing a local therapeutic effect, thus avoiding the systemic toxicity due to the direct parenteral administration of the drug. In this regard, the porous polymeric matrix can also help due to the fast diffusion of some molecular species through the pores. In one of the examples provided below, the porous polymeric matrix has positively charged groups. The prodrug is a Pt(IV) complex prodrug which is electrostatically immobilized in the matrix due to the positive charge of the matrix and the negative charges of the succinate ligands in the prodrug . Once the reduction starts, due to the incident light stimuli, the Pt(IV) complex is converted into a Pt(II) positively charged complex. This Pt(II) complex is repelled by the positively charged groups of the matrix, giving rise to its diffusion through the matrix out from the catalytic system.

Therefore, the catalytic system is useful in the therapeutic field, but also in the diagnostic field, wherein the substrate can be, for instance, the analyte to be reduced/oxidized in an isolated test sample.

In view of the above, in a third aspect the present invention provides a catalytic system as defined in the first aspect of the invention for use in therapy or diagnostics.

In a fourth aspect the present invention provides a catalytic system as defined in the first aspect of the invention, for use in the treatment or prevention of cancer. This aspect can alternatively be formulated as the use of a catalytic system as defined in the first aspect of the invention in the manufacture of a drug for the treatment or prevention of cancer. This aspect can also be alternatively formulated as a method for treating or preventing cancer, the method comprising administering a therapeutically effective amount of the catalytic system as defined in the first aspect of the invention to a subject in need thereof.

In a fifth aspect the present invention provides a pharmaceutical or cosmetical composition comprising a catalytic system as defined in the first aspect of the invention comprising a therapeutic or cosmetical effective amount of an oxidable or reducible cosmetical or pharmaceutical substance, and one or more therapeutic or cosmetical excipients and/or carriers.

In a sixth aspect the present invention provides a device, such as a medical device or reactor, comprising the catalytic system as defined in the first aspect of the invention.

In a seventh aspect the present invention provides a catalytic system as defined in the first aspect of the invention for use in a method for treating or preventing cancer, the method comprising the steps of administering the catalytic device as defined in the sixth aspect of the invention, or the pharmaceutical composition as defined in the fifth aspect of the invention, and applying an external stimuli selected from light, temperature, a REDOX cofactor, and a combination thereof. This aspect can alternatively be formulated as a method for treating or preventing cancer, the method comprising administering a therapeutically effective amount of the catalytic system as defined in the first aspect of the invention, and (b) applying an external stimulus selected from light, temperature, a REDOX cofactor, and a combination thereof.

In an eighth aspect the present invention provides a kit of parts, comprising:

i) a compartment "A" comprising a porous polymeric matrix as defined in the first aspect of the invention; a compartment "B" comprising the catalyst as defined in the first aspect of the invention; and, optionally, a compartment "C" comprising a substrate to be reduced or oxidized (to be used, for instance, as control); or, alternatively,
ii) a compartment "D" comprising the catalytic system according to the first aspect of the invention, and, optionally, a

compartment "E" comprising a substrate to be reduced or oxidized (to be used, for instance, as control),

together with instructions for its use

In a final aspect, the present invention provides an ex *vivo* method for performing a REDOX catalytic reaction of a substrate capable of being reduced or oxidized by a isoalloxazine-based catalyst, the method comprising (a) contacting the substrate with the catalytic system as defined in the first aspect of the invention; and (b) applying an external stimuli selected from light, temperature, a REDOX cofactor, differential voltage applied between electrodes, and a combination thereof;

## Brief Description of Drawings

Fig. 1 corresponds to SEM micrographs showing the AGM-D structure and porosity under different conditions. A and D for the agarose matrix alone, B and E for the catalytic system including both the Pt(IV) complex as well as the FMN catalyst at dark, and C and F for the catalytic system including both the Pt(IV) complex as well as the FMN catalyst once irradiated (5 min , 460 nm, 6 mW $cm^{-2}$).

Fig.2 shows the elution of FMN from the AGM-D matrix ("Y" axis, expressed in nmol·of FMN per g of AGM-D matrix) after incubation ("X" axis) with phosphate saline buffer (PBS: 50 mM sodium phosphate, 150 mM NaCl at pH 7,2). In the "X" axis, I.L.= Initial load and corresponds to the amount of FMN bound to the polymeric matrix after the immobilization process; "PBS"= after adding PBS 1X corresponds to the amount of FMN that remains bound to the polymeric matrix after its incubation with PBS 1X.

Fig.3 (a) represents the Pt(IV) substrate conversion in 1-FMN@AGM-D ("Y" axis, "S.C.", expressed in %) with respect to the time ("X" axis, "t", expressed in minutes) of exposure to a low-energy light (460 nm, 6 mW $cm^{-2}$), indicating that after 5 minutes 100% of substrate conversion (from Pt(IV) to Pt(II)) was achieved. For each time point, quantification of substrate conversion was achieved after washing the samples with 1 M NaCl; (b) represents the concentration ("Y" axis, "C", expressed in mM) of the Pt(IV) substrate (1) and Pt(II) drug released (S) (in the "X" axis) with (light grey bar) and without (dark grey bar) 1 M NaCl washing in the dark (I) and after light irradiation (II). The substrate conversion and Pt(II) drug release plots were drawn using $^1$H NMR (a) and UPLC-MS (b) data.

## Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the present invention, any ranges given include both the lower and the upper end-points of the range.

The present invention provides a catalytic system comprising a porous polymeric matrix and an isoalloxazine-based catalyst.

In the present invention the term "porous polymeric matrix" refers to a continuous layer or particulated material of small size, made of one or more types of different polymeric chains, either synthetic or natural. This porous polymeric matrix can be used self-supported or coating another material.

In one embodiment, optionally in combination with any of the embodiments provided above or below, the porous polymeric matrix is made from polymeric chains made of one or more monomers having a logP value equal or lower than 0 (i.e., the porous polymeric matrix has hydrophilic or amphiphilic nature). Quantitatively, the hydrophobic/hydrophilic nature of the monomers may be determined according to the log P of the particular monomers, where P, also known as Pow and Kow, is sometimes referred to as the octanol-water partition coefficient.

The partition coefficient, abbreviated P, is defined as a particular ratio of the concentrations of a monomer between the two solvents (a biphase of liquid phases), and the logarithm of the ratio is thus log P. Log P values are well known and are determined according to a standard test that determines the concentration of monomer in a water/1-octanol separated mixture:

$$\text{LogP} = \log10\ (\text{Partition Coefficient})\text{Partition Coefficient, P} = [\text{organic}]/[\text{aqueous}]$$

where [] indicates the concentration of solute in the organic and aqueous partition. A negative value for logP means the compound has a higher affinity for the aqueous phase (it is more hydrophilic); when logP = 0 the compound is equally partitioned between the lipid and aqueous phases; a positive value for logP denotes a higher concentration in the organic phase (i.e., the compound is more lipophilic).

There are also computer programs, commercially available, as well as on various internet sites, that will estimate the log P values for particular monomers.

Accordingly, monomers employed in preparing the porous polymeric matrix which make the hydrophilic polymer network of the invention will typically have a log P value of less than about 1 or 0.5. For example, the following hydrophilic monomers have the following log P values: acrylic acid, about 0.35; 2-methoxyethylacrylate, about 0.45; and 2-hydroxyethyl-methacrylate, about 0.47. Other hydrophilic monomers and their log P values include, but are not limited to, acrylamide (about -0.67), 2-hydroxyethylacrylate (about -0.21), acrylic acid (0.35), methacrylic acid (0.93), N,N-dimethylacrylamide (-0.13), quaternized dimethylaminoethyl methacrylate, methacrylamide (-0.26), maleic acid (-0.48), maleic anhydride and its half esters, crotonic acid (0.72), itaconic acid (-0.34), acrylamide (-0.67), acrylate alcohols, hydroxyethyl methacrylate, diallyldimethyl ammonium chloride, vinyl ethers (such as methyl vinyl ether), maleimides, vinyl pyridine, vinylimidazole (0.96), other polar vinyl heterocycles, styrene sulfonate, allyl alcohol (0.17), vinyl alcohol (such as that produced by the hydrolysis of vinyl acetate after polymerization), salts of any acid or amine listed above, as well as mixtures thereof. Among the saccharides, galactose (either alpha or beta) has a logP value of about - 2.6, mannitol of about - 2.2, glucose of about -2.4/-3.6, for instance. In one embodiment of the first aspect of the invention, the one or more monomers forming the porous polymeric matrix have a logP value lower than 0. In one embodiment of the first aspect of the invention, the one or more monomers forming the porous polymeric matrix have a logP value lower from -5 to 0, from -4 to -1, from -3.5 to -1.5.

The synthetic polymers which may be utilized in the present invention may be non-biodegradable or biodegradable synthetic water insoluble polymers. The biodegradable polymers may be polyesters, polyanhydrides, or polyortho esters. Representative polyesters include poly(lactic acid), poly(glycolic acid), and poly(lactic acid-co-glycolic acid), poly(lactide) [p(LA)], poly(glycolide) [p(GA)], poly(lactide-co-glycolide) [p(LGA)], polycaprolactone and poly(lactide-co-caprolactone). Representative polyortho esters include the polyortho esters prepared by condensation polymerization of 3,9-diethylidene-2,4,8,10-tetraoxaspiroundecane (DETOSU) and the diols containing trans-cyclohexanedimethanol, triethylene glycol or N-methyldiethanolamine, and their copolymers with p(LGA) or polyethylene glycol; the preferred polyortho esters are their copolymer containing p(LGA). Other biodegradable polymers include polyamides, such as polypeptides; poly(butyric acid), poly(valeric acid), and their copolymers, polyalkylene glycols such as poly(ethylene glycol), polyalkylene oxides such as poly(ethylene oxide). Non-biodegradable polymers include polyalkylene terepthalates such as poly(ethylene terephthalate), polyvinyl alcohols, polyvinyl halides such as poly(vinyl chloride), polyvinylpyrrolidone, polysiloxanes, poly(vinyl acetate), polystyrene, polyurethanes, polycarbonates, polyalkylenes such as polyethylene and polypropylene, and co-polymers thereof. Polymers of acrylic acid, methacrylic acid or copolymers or derivatives thereof including, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate) (jointly referred to herein as "polyacrylic acids"), and blends thereof. As used herein, "derivatives" include polymers having substitutions, additions of chemical groups, for example, alkyl, alkylene, hydroxylations, oxidations, and other modifications routinely made by those skilled in the art.

Representative natural polymers and their derivatives include the following: proteins such as albumin and polysaccharides such as alginates, pectins, hyaluronic acid, carrageenans, agarose, agaropectins, amyloses, the amylopectins, arabino-galactans, chitosan, tragacanth, gum arabic, guar gum, xanthan, dextrans, collagen and gelatinsp, chitosan, hyaluronate esters, polyhydroxybutyrate, cellulose and derivativized celluloses such as alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, and cellulose sulphate sodium salt (jointly referred to herein as "synthetic celluloses"). In one embodiment the porous polymeric matrix comprises a natural polymer, particularly a polysaccharide. In another embodiment the porous polymeric matrix consists of a natural polymer, particularly a polysaccharide.

In one embodiment of the first aspect of the invention, optionally in combination with any of the embodiments provided above or below, the porous polymeric matrix comprises a crosslinked polymer. In the present invention the term "crosslinked polymer" is used in a wide sense, and refers to any type of covalent polymer network, dynamic or reversible covalent polymer networks or supramolecular polymer networks. Illustrative non-limitative examples of covalent bonds, useful in linking polymer chains, are: ether (-C(=O)-), thioether (-S(=O)-), ester (-O-C(=O)-), amide ( C(=O)-NH-), disulfide urethane (-N-C(=S)-S-), thiourethane (-N-C(=O)-S-), and borate (-O-B-O-) bonds. The crosslink between two polymeric chains can be performed by chemical reactions that are initiated by heat, pressure, change in pH, or radiation. For example, mixing of an unpolymerized or partially polymerized matrix with specific chemicals called crosslinking reagents

results in a chemical reaction that forms crosslinks. Cross-links are selected on the basis of their chemical reactivities (i.e., specificity for particular function groups) and other chemical properties that affect their behavior in different applications: (a) Chemical specificity refers to the reactive target(s) of the crosslink reactive ends. A general consideration is whether the reagent has the same or different reactive groups at either end; (b) Spacer arm length refers to the molecular span of a crosslinking (i.e., the distance between conjugated molecules). A related consideration is whether the arm is cleavable (i.e., whether the linkage can be reversed or broken when desired); and (c) Spontaneously reactive or photoreactive groups in a crosslinking affect whether it reacts as soon as it is added to a sample or can be activated at a specific time by exposure to UV light. In another embodiment of the first aspect of the invention, the polymer has a degree of crosslinking from 0.5 to 50%. The "degree of crosslinking" expressed in %, means the % of polymeric chains crosslinked with respect to the total of polymeric chains forming the polymer. This degree of cross-linking can be determined through well-known techniques such as Scanning Electronic Microscope (SEM) for measuring the density of cross-linking in films and membrane. Also, the elemental analysis for determining the percentage of cross-linking in bulk mass. In another embodiment, the degree of cross-linking is from 1 to 20% or from 5 to 15%. In another embodiment of the first aspect of the invention the polymer is agarose and has a degree of cross-linking from 1 to 20% or from 5 to 15%.

In another embodiment of the first aspect of the invention, optionally in combination with any of the embodiments provided above or below, the "porous polymeric matrix" comprises sugar-based polymeric chains wherein at least a 50%, at least a 60%, at least a 70%, at least a 80%, at least a 90%, at least a 95% or the 100% of the monomers forming the polymeric chains have a Log P value equal or lower than 0. In another embodiment of the first aspect of the invention, optionally in combination with any of the embodiments provided above or below, the "porous polymeric matrix" consists of sugar-based polymeric chains wherein at least a 50%, at least a 60%, at least a 70%, at least a 80%, at least a 90%, at least a 95% or the 100% of the monomers forming the polymeric chains have a Log P value equal or lower than 0. In another embodiment of the first aspect of the invention, optionally in combination with any of the embodiments provided above or below, the "porous polymeric matrix" comprises a sugar-based polymer comprising a saccharide monomer having a Log P value equal or lower than 0, the saccharide monomer representing at least a 50%, at least a 60%, at least a 70%, at least a 80%, at least a 90%, at least a 95% or the 100% of the polymer. In another embodiment of the first aspect of the invention, optionally in combination with any of the embodiments provided above or below, the "porous polymeric matrix" consists of sugar-based polymeric chains comprising a saccharide monomer having a Log P value equal or lower than 0, the saccharide monomer representing at least a 50%, at least a 60%, at least a 70%, at least a 80%, at least a 90%, at least a 95% or the 100% of the polymeric chains. In another embodiment of the first aspect of the invention, optionally in combination with any of the embodiments provided above or below, the "porous polymeric matrix" consists of sugar-based polymeric chains comprising galactose, this saccharide representing at least a 50%, at least a 60%, at least a 70%, at least a 80%, at least a 90%, at least a 95% or the 100% of the polymeric chain. In another embodiment, optionally in combination with any of the embodiments provided above or below, the porous polymeric matrix consists of polymeric chains of agarose, such as agarose beads, particularly microbeads. In another embodiment, optionally in combination with any of the embodiments provided above or below, the porous polymeric matrix consists of crosslinked polymeric chains of agarose, such as crosslinked agarose beads, particularly microbeads. In another embodiment, optionally in combination with any of the embodiments provided above or below, the porous polymeric matrix consists of crosslinked polymeric chains of agarose, such as crosslinked agarose beads, particularly microbeads, with a degree of crosslinking from 1 to 20%, particularly from 5 to 15%. In another embodiment, optionally in combination with any of the embodiments provided above or below, the porous polymeric matrix is in the form of a hydrogel. In another embodiment the porous polymeric matrix is a hydrogel with agarose polymeric chains in the form of beads, such as microbeads.

Similarly, the term "porous polymeric matrix" also embraces inorganic porous materials with the ability to anchor both catalyst and substrates. Illustrative non-limitative examples are silica, titania, alumina, metal phosphates (i.e copper phosphate, zinc phosphate) and calcium carbonates.

The pore size is comprised between 1 and 1000 nm. The "pore size" is understood as the radius of a pore which is measured by well-known routine techniques such as Scanning Electron Microscopy (SEM). In one embodiment, the pore size is comprised from 20 to 800 nm, from 40 to 600 nm, from 50 to 400 nm, from 60 to 300 nm or from 70 to 200 nm.

In one embodiment, optionally in combination with any of the embodiments provided above or below, the polymeric matrix has a porosity degree of at least a 50%, at least a 60%, at least a 70%, at least a 80% or at least a 90%. The porosity degree determines the loading of catalyst and substrate: the higher the porosity degree, the higher the loading. The porosity degree can be determined as disclosed by Pluen and colleagues (cf. Pluen A. et al., 1999), using the equation:

$$\text{porosity degree or void volume (\%)} = 100-(100 \times \phi)$$

where $\phi$ is the volume fraction occupied by the fibers forming the polymeric matrix and it is defined as:

$$\phi = C/(\rho\omega);$$

where c is the concentration of the material forming the polymeric matrix expressed in g $\times$ $mL^{-1}$ units, $\rho$ is the material density with units g $\times$ $mL^{-1}$, and $\omega$ is the mass fraction of the material in a fiber.

In one embodiment, optionally in combination with any of the embodiments provided above or below, the porosity of the polymeric matrix is of at least a 90% to assure high loadings of catalyst and metal substrates, as well as a higher contact between them. In one embodiment, optionally in combination with any of the embodiments provided above or below, the porosity degree of the polymeric matrix is from 90% to 98%. In one embodiment, optionally in combination with any of the embodiments provided above or below, the porosity degree of the polymeric matrix is of about 90%, 91%, 92%, 93%, 94%, 95%, 96%, 97%, or 98%.

In one embodiment, optionally in combination with any of the embodiments provided above or below, the pores have an size from 100 to 1000 nm and the porous matrix has a porosity degree from 50 to 98%, from 60 to 98%, from 70 to 98%, from 80 to 98% or from 90 to 98%.

As it has been stated above, the catalytic system of the invention is based on the non-covalent interaction between the catalyst and the polymeric matrix. A non-covalent interaction differs from a covalent bond in that it does not involve the sharing of electrons, but rather involves more dispersed variations of electromagnetic interactions between molecules or within a molecule. The chemical energy released in the formation of non-covalent interactions is typically on the order of 1-5 kcal/mol.

The term "non-covalent reversible interaction" (hereinafter also referred to as "reversible immobilization") between the catalyst and the porous polymeric matrix, means a reversible anchoring of the catalyst in the polymeric matrix by non-covalent interactions (such as electrostatic interactions, hydrophobic interactions or hydrogen bonds). The reversibility is due to the chemical nature of the $R_3$-functionalized catalyst combined with the excess of anchoring spots and the electronic environment provided by $R_1$ groups or alternatively by $R'_1$ and $R_2$ groups. In one embodiment of the first aspect, the $R_1$ or $R_2$ functional groups are at a concentration, expressed in terms of $\mu$moles of functional groups per gram of porous polymeric matrix, is equal to or higher than 5, 10, 15, 20, 25, 30, 35, 40, 45, 50, 55, 60, 65, 70, 75, 80, 85, 90, 95, 100, 200, 300, 400 or 500 $\mu$mol $\times$ $g^{-1}$ . In another embodiment, optionally in combination with any of the embodiments provided above or below, $R_1$, and $R_2$ are at a molar concentration excess with respect to the molar concentration of catalyst, being at a molar concentration from 50 to 500 $\mu$mol $\times$ $g^{-1}$, such as 100 $\mu$mol $\times$ $g^{-1}$.

The concentration of functional groups $R_1$, $R'_1$ and $R_2$ are determined by indirect colorimetric quantification comprising determining the amount of epoxy groups in the matrix before and after the functionalization with for example 2,2'-diethyl aminoethanol (for more details see example).

The porous polymeric matrix is functionalized with groups $R_1$ or, alternatively, with groups $R'_1$ and $R_2$. These groups are covalently bound to the porous polymeric matrix and are responsible for providing the appropriate electronic environment, for the correct catalytic effect, and the anchoring of the catalyst. The selection of one or other $R_1$ or $R'_1$ and $R_2$ functional groups greatly depends on the interaction to be induced in the catalytic system, the latter mainly depending on the nature of both the catalyst and the substrate.

The selection of the functional groups forming part of the matrix forms part of the common general knowledge of the skilled person in the art. Table 1 provides some examples:

**Table 1**

| Catalyst ($R_3$) | Polymer matrix ($R_1$) | Type of interaction |
|---|---|---|
| Negatively charged phosphate groups of FMN and FAD | Positively charged groups in the functionalized polymer matrix such as both aromatic and aliphatic primary, secondary and tertiary amino groups. | Ionic electrostatic |
| Positively charged flavins | Polymeric matrix functionalized with carboxylates, sulfonates, phosphonates, silanols... | Ionic electrostatic |
| OH groups of the ribityl chain of flavin | Polymeric matrix functionalized with carboxylates, fluorines (i.e CF3) acids, alcohols, carbonyls, sulfonates, phosphonates and amide groups | H-bond electrostatic |

(continued)

| Catalyst ($R_3$) | Polymer matrix ($R_1$) | Type of interaction |
|---|---|---|
| C=O and NH groups on the isoalloxazine moiety of the flavin catalyst<br>In the case of other specific flavins (see below structures) the isoalloxazine rings bear also groups such as OH, COOH and SH, $PO_4$- the could afford the same type of interaction | Polymeric matrix functionalized with charged amino groups, carboxylic acids and alcohols | H-bond electrostatic |
| Isoalloxazine moiety of flavin | Polymeric matrix functionalized with aromatic groups as benzyl, phenyl rings and/or pseudoaromatic heterocycles | Hydrophobic (p-p) |
| Isoalloxazine moiety functionalized with alkyls and/or aromatic chains (position N3 and N10) | Polymeric matrix functionalized with saturated and unsaturated alkyl chains | Hydrophobic |

In one embodiment of the first aspect of the invention, optionally in combination with any of the embodiments provided above or below:

- when $R_3$ is an hydroxyl, aldehyde, ketone, ether, carboxylic acid, carboxylate, thiol, ester, amide, nitrile, sulfide, alkyl halide, nitro compound, sulfonic acid, sulfonate, sulfinic acid, sulfinate, silanol, azide, cyanate, isocyanate, thiocyanate, thiocarboxylic acid, phosphate or phosphine, then the porous polymeric matrix comprises $R_1$ functional groups which are selected from an amine and a phosphine; or alternatively,

- when $R_3$ is an amine, imine, imide, nitrate, pyridine or oxyme, then the porous polymeric matrix comprises $R_1$ functional groups which are selected from: carboxylic acid, sulfinic acid, sulfonic acid, boronic acid, hydroxyl, geminal diol, vicinal diol, 1,3-diol, thiol and silanol; or, alternatively,

- when $R_3$ is ($C_4$-$C_{30}$) alkyl, ($C_4$-$C_{30}$)alkenyl or aryl, then the porous polymeric matrix comprises $R'_1$ and $R_2$ functional groups, wherein the $R'_1$ functional groups are selected from amine, sulfonic, sulfinic, hydroxyl, aldehyde, ketone, carboxylic acid, thiol, ester, amide, nitrile, sulfide, alkyl halide, nitro compound, azide, cyanate, isocyanate, thiocyanate, thiocarboxylic acid, phosphate, phosphine, phosphine oxides and silanol; and $R_2$ functional groups are selected from ($C_4$-$C_{30}$) alkyl, ($C_4$-$C_{30}$)alkenyl and aryl; or, alternatively,

- when $R_3$ is a cis diol, or hydroxamic acid, then the porous polymeric matrix comprises $R_1$ functional groups which are selected from boronic acid, and amide; or, alternatively,

- when $R_3$ is a polypeptide, then the porous polymeric matrix comprises $R_1$ functional groups which are selected from amine, carboxyl, thiol, aldehyde and epoxide groups.

The term "functional group" when referred to $R_1$, $R'_1$, $R_2$ and $R_3$, and unless otherwise specified, refers to the nature of chemical moiety which is essentially responsible for the reversible non-covalent interaction and/or electron behaviour, depending on the case. For example, if it is envisaged that $R_1$ can be an "amine functional group", this means that the porous polymeric matrix comprises amine groups, of any nature (primary, secondary or tertiary), either as such (i.e., the matrix is functionalized with a particular primary, secondary or tertiary amine) or it is comprised in a larger molecule (such as alkyls, alkenyls, alkynyls, ring systems, etc) including as functional group an amine functional group (either primary, secondary or tertiary). If $R_1$ means a carboxylic acid functional group, it means that the porous polymeric matrix comprises either -COOH as such or larger molecules including one or more -COOH. And the same with the other substituents contemplated below for each one of the functional groups.

In one embodiment, optionally in combination with any of the embodiments provided above or below, the non-covalent interaction between the porous matrix and the catalyst is of electrostatic type, particularly ionic electrostatic, which means that both, the porous polymeric matrix (functionalized with $R_1$ or, alternatively, $R'_1$ and $R_2$ groups) and the catalyst (functionalized with $R_3$ functional groups) are oppositely charged.

In one embodiment of the catalytic system of the invention, optionally in combination with any of the embodiments provided above or below, the non-covalent interaction between the porous matrix and the catalyst is of electrostatic type, particularly ionic electrostatic, the catalyst is negatively charged, whereas the porous polymeric matrix is positively charged. In the demonstration herein presented, the catalyst is negatively charged due to phosphates, whereas the matrix

is functionalized with positively charged amine groups (tertiary amines). These electrostatic pairs can be expanded to other charged amine groups (primary, secondary, tertiary) both aliphatic and aromatic as well as cyclic ones, ammonium, imidazoles, pyridines and pyrrols. Other reversible interactions thermodynamically governed by an association/dissociation equilibrium are also contemplated in the invention according to the functionalization examples provided in Table 1 above.

Alternatively, in another embodiment of the catalytic system of the invention, optionally in combination with any of the embodiments provided above or below, the non-covalent interaction between the porous matrix and the catalyst is of electrostatic type, particularly ionic electrostatic, and the catalyst is positively charged, whereas the porous polymeric matrix displays negative charges.

In another embodiment of the first aspect of the invention, the non-covalent interaction between the porous matrix and the catalyst is of electrostatic type, particularly ionic electrostatic, and the porous polymeric matrix is covalently functionalized with $R_1$ functional groups which are the same or different and are selected from: $-R_4N^{(+)}R_6R_5$, $-P^{(+)}R_7R_8R_9R_{10}$, -COO-, $-SO_{3-}$, -SO-, $-B(OH)_{2-}$, $-R_{11}$-COO-, $-R_{12}-SO_{3-}$, $-R_{13}$-SO-, and $-R_{12}-B(OH)_{2-}$; wherein

$R_4$ to $R_{10}$ are the same or different and are independently selected from the group consisting of -H; $(C_1-C_{20})$ alkyl optionally substituted with one or more groups selected from $NR_{15}R_{16}$, nitro, hydroxyl, halogen, $-O-R_{35}$, $(C_1-C_3)$ haloalkyl, $(C_3-C_7)$ cycloalkyl, and aryl; $(C_2-C_{20})$ alkenyl optionally substituted with one or more groups selected from $NR_{17}R_{18}$, nitro, hydroxyl, halogen, $-O-R_{36}$, $(C_1-C_3)$haloalkyl, $(C_3-C_7)$ cycloalkyl and aryl; $(C_1-C_{20})$ alkynyl optionally substituted with one or more groups selected from $NR_{19}R_{20}$, nitro, hydroxyl, halogen, $-O-R_{37}$, $(C_1-C_3)$haloalkyl, $(C_3-C_7)$ cycloalkyl and aryl; a $(C_3-C_7)$ cycloalkyl optionally substituted with one or more groups selected from $NR_{21}R_{22}$, nitro, hydroxyl, halogen, $-O-R_{38}$, $(C_1-C_3)$haloalkyl, $(C_2-C_8)$alkenyl and $(C_2-C_8)$alkynyl; a $(C_5-C_6)$ aromatic ring optionally substituted with one or more groups selected from $NR_{23}R_{24}$, nitro, hydroxyl, halogen, $-OR_{39}$, $(C_1-C_3)$ haloalkyl, $(C_2-C_8)$alkenyl and $(C_2-C_8)$alkynyl; or, alternatively, $R_4$, $R_5$ and $R_6$ form with the N atom a $(C_5-C_6)$ saturated, partially saturated or aromatic ring system;

$R_{11}$ a $R_{14}$ are selected from $(C_1-C_{20})$ alkyl optionally substituted with one or more groups selected from $NR_{25}R_{26}$, nitro, hydroxyl, halogen, $-O-R_{39}$, $(C_1-C_8)$alkyl, $(C_1-C_8)$haloalkyl, $(C_2-C_8)$alkenyl, $(C_2-C_8)$haloalkenyl, $(C_3-C_7)$ cycloalkyl, and aryl; $(C_2-C_{20})$ alkenyl optionally substituted with one or more groups selected from $NR_{27}R_{28}$, nitro, hydroxyl, halogen, $-O-R_{40}$, $(C_1-C_8)$alkyl, $(C_1-C_3)$haloalkyl, $(C_2-C_8)$alkenyl, $(C_2-C_8)$haloalkenyl, $(C_3-C_7)$ cycloalkyl, and aryl; $(C_2-C_{20})$ alkynyl optionally substituted with one or more groups selected from $NR_{29}R_{30}$, nitro, hydroxyl, halogen, $-O-R_{41}$, $(C_1-C_8)$alkyl, $(C_1-C_8)$haloalkyl, $(C_3-C_7)$ cycloalkyl, and aryl; a $(C_3-C_7)$ cycloalkyl optionally substituted with one or more groups selected from $NR_{31}R_{32}$, nitro, hydroxyl, halogen, $-O-R_{42}$, $(C_1-C_8)$alkyl, $(C_1-C_8)$haloalkyl, $(C_2-C_8)$alkenyl, $(C_2-C_8)$haloalkenyl, $(C_2-C_8)$alkynyl, and $(C_2-C_8)$haloalkynyl; a $(C_5-C_6)$ aromatic ring optionally substituted with one or more groups selected from $NR_{32}R_{34}$, nitro, hydroxyl, halogen, $-OR_{43}$, $(C_1-C_8)$alkyl, $(C_1-C_8)$haloalkyl, $(C_2-C_8)$alkenyl, $(C_2-C_3)$haloalkenyl, $(C_2-C_8)$alkynyl, and $(C_2-C_8)$haloalkynyl;

$R_{15}$ to $R_{34}$ are the same or different and are selected from the group consisting of -H, $(C_1-C_{10})$alkyl, $(C_1-C_{10})$haloalkyl, $(C_2-C_{10})$alkenyl, $(C_2-C_{10})$alkynyl, $(C_3-C_7)$ cycloalkyl, and aryl; and

$R_{35}$ to $R_{43}$ are selected from $(C_1-C_{10})$alkyl, $(C_1-C_{10})$haloalkyl, $(C_2-C_{10})$alkenyl, $(C_2-C_{10})$alkynyl, $(C_3-C_7)$ cycloalkyl, and aryl.

In another embodiment, optionally in combination with any of the embodiments provided above or below, the non-covalent interaction between the porous matrix and the catalyst is of electrostatic type, particularly ionic electrostatic, the porous polymeric matrix is covalently functionalized with $R_1$ functional groups which are the same or different and are selected from: $-R_4N^{(+)}R_6R_5$, and $-P^{(+)}R_7R_8R_9R_{10}$, being $R_4$ to $R_{10}$ as defined above. In this embodiment, the catalyst has $R_3$ functional groups negatively charged.

In another embodiment, optionally in combination with any of the embodiments provided above or below, $R_1$ represents $-R_4N^{(+)}R_5R_6$ and $R_4$ to $R_6$ are as defined in claim 3, particularly selected from the group consisting of "$R_1$" represents $-R_4N^{(+)}R_5R_6$ and $R_4$ to $R_6$ are as defined in claim 3, particularly selected from the group consisting of

· $(C_1-C_{20})$ alkyl optionally substituted with one or more groups selected from $NR_{25}R_{26}$, nitro, hydroxyl, halogen, $-O-R_{39}$, $(C_1-C_8)$alkyl, $(C_1-C_8)$haloalkyl, $(C_2-C_8)$alkenyl, $(C_2-C_3)$haloalkenyl, $(C_3-C_7)$ cycloalkyl, and aryl;
· $(C_2-C_{20})$ alkenyl optionally substituted with one or more groups selected from $NR_{27}R_{28}$, nitro, hydroxyl, halogen, $-O-R_{40}$, $(C_1-C_8)$alkyl, $(C_1-C_8)$haloalkyl, $(C_2-C_8)$alkenyl, $(C_2-C_3)$haloalkenyl, $(C_3-C_7)$ cycloalkyl, and aryl;
· $(C_2-C_{20})$ alkynyl optionally substituted with one or more groups selected from $NR_{29}R_{30}$, nitro, hydroxyl, halogen, $-O-R_{41}$, $(C_1-C_8)$alkyl, $(C_1-C_8)$haloalkyl, $(C_3-C_7)$ cycloalkyl, and aryl;

particularly $(C_1-C_{20})$ alkyl optionally substituted with one or more groups selected from $NR_{25}R_{26}$, nitro, hydroxyl, halogen, $-O-R_{39}$, $(C_1-C_8)$alkyl, $(C_1-C_8)$haloalkyl, $(C_2-C_8)$alkenyl, $(C_2-C_3)$haloalkenyl, $(C_3-C_7)$ cycloalkyl, and aryl.

In another embodiment, optionally in combination with any of the embodiments provided above or below, $R_1$ represents $-R_4N^{(+)}R_5R_6$, wherein $R_4$ to $R_6$ are the same and represent a $(C_1-C_{20})$alkyl, $(C_2-C_{20})$alkenyl or $(C_2-C_{20})$alkynyl, particularly $(C_1-C_{20})$alkyl.

The term $(C_1-C_{20})$alkyl refers to a saturated straight or branched alkyl chain having from 1 to 20 carbon atoms. The term $(C_1-C_8)$alkyl refers to a saturated straight or branched alkyl chain having from 1 to 8 carbon atoms. The term $(C_4-C_{30})$alkyl refers to a saturated straight or branched alkyl chain having from 4 to 30 carbon atoms. Illustrative non-limitative examples are: methyl, ethyl, propyl, isopropyl, butyl, isobutyl, sec-butyl, tert-butyl, n-pentyl, neo-pentyl and n-hexyl.

The term $(C_1-C_8)$haloalkyl refers to a group resulting from the replacement of one or more hydrogen atoms from a $(C_1-C_8)$alkyl group with one or more, preferably from 1 to 8, halogen atoms, which can be the same or different. Examples include, among others, trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, and nonafluorobutyl.

The term $(C_2-C_{20})$alkenyl refers to a saturated straight, or branched alkyl chain containing from 2 to 20 carbon atoms and also containing one or more double bonds. The term $(C_2-C_3)$alkenyl refers to a saturated straight, or branched alkyl chain containing from 2 to 8 carbon atoms and also containing one or more double bonds. The term $(C_4-C_{30})$alkenyl refers to a saturated straight, or branched alkyl chain containing from 4 to 30 carbon atoms and also containing one or more double bonds. Illustrative non-limitative examples are ethenyl, propenyl, butenyl, 1-methyl-2-buten-1-yl, and the like.

The term $(C_2-C_8)$haloalkenyl refers to a saturated straight or branched alkyl chain having from 2 to 8 carbon atoms, also containing one or more double bonds, and wherein one or more of the carbon atoms is/are bound to one or more halogen atoms.

The term $(C_2-C_{20})$alkynyl refers to a saturated straight, or branched alkyl chain containing from 2 to 20 carbon atoms and also containing one or more triple bonds. The term $(C_2-C_3)$alkynyl refers to a saturated straight, or branched alkyl chain containing from 2 to 8 carbon atoms and also containing one or more triple bonds Examples include, among others, ethynyl, 1-propynyl, 2-butynyl, 1,3-butadinyl, 4-pentynyl, and 1-hexynyl.

The term $(C_2-C_8)$haloalkynyl refers to a saturated straight, or branched alkyl chain containing from 2 to 8 carbon atoms and also containing one or more triple bonds, wherein one or more carbon atoms is/are bound to one or more halogen atoms.

The term $(C_3-C_7)$cycloalkyl refers to a saturated cyclic alkyl from 3 to 8 carbon atoms. Examples include: cyclopropyl, cyclobutyl, cyclopentyl, cyclohexyl, cycloheptyl, cyclopropenyl, cyclobutenyl, and cyclopentenyl and cycloheptenyl.

The term "aryl" refers to a known aromatic ring system comprising 5 or 6 members selected from CH, $CH_2$, NH, N, O, and S. Illustrative non-limitative examples are imidazoles, pyridines and pyrroles. When the aryl group is made of 5 or 6 members selected from CH and $CH_2$, then it is also referred as a "$(C_5-C_6)$ aromatic ring"; and when the aryl group is made of 5 or 6 members wherein at least one of them is NH, N, O or S, then it can also be referred as "$(C_5-C_6)$ heteroaromatic ring".

In another embodiment of the first aspect of the invention, optionally in combination with any of the embodiments provided above or below, $R_3$ functional groups form an electrostatic interaction (i.e., non-covalent reversible immobilization) with the $R_1$ or $R_2$ groups of the porous polymeric matrix. In another embodiment of the first aspect of the invention, optionally in combination with any of the embodiments provided above or below, $R_3$ are negatively charged functional groups and form an electrostatic interaction with $R_1$ or $R_2$ groups of the porous polymeric matrix, wherein $R_1$ or $R_2$ which are positively charged functional groups. In another embodiment of the first aspect of the invention, optionally in combination with any of the embodiments provided above or below, the $R_3$ positively charged functional groups and form an electrostatic interaction with the $R_1$ or $R_2$ groups of the porous polymeric matrix, wherein $R_1$ or $R_2$ are negatively charged functional groups. In another embodiment, optionally in combination with any of the embodiments provided above or below, the $R_3$ are negatively charged functional groups and form an electrostatic interaction with $R_1$ groups of the porous polymeric matrix which corresponds to $-R_4N^{(+)}R_5R_6$, being $R_4$ to $R_6$ as defined above. In another embodiment, optionally in combination with any of the embodiments provided above or below, the $R_3$ functional groups form an electrostatic interaction with "$R_1$" groups of the porous polymeric matrix represented by $-R_4N^{(+)}R_5R_6$, being $R_4$ to $R_6$ are the same. In another embodiment, the "$R_3$" functional groups form an electrostatic interaction with the one or more $R_1$ groups of the porous polymeric matrix represented by $-R_4N^{(+)}R_5R_6$, wherein $R_4$ to $R_6$ are the same and represent a $(C_1-C_{20})$alkyl, $(C_2-C_{20})$alkenyl or $(C_2-C_{20})$alkynyl, particularly $(C_1-C_{20})$alkyl. In another embodiment, optionally in combination with any of the embodiments provided above or below, the $R_3$ functional groups form an electrostatic interaction with $R_1$ groups of the porous polymeric matrix represented by $-R_4N^{(+)}R_5R_6$, wherein $R_4$ to $R_6$ are as defined above, and $R_3$ are negatively-charged functional groups, the same or different, selected from phosphate, sulphate, -COO-, $-SO_{3-}$, -SO-, $-B(OH)_{2-}$, $R''_{11}$-COO-, $-R''_{12}-SO_{3-}$, $-R''_{13}$-SO-, and $-R''_{14}-B(OH)_{2-}$, wherein $R''_{11}$, $R''_{12}$, $R''_{13}$ and $R''_{14}$ are the same or different and are

independently selected from the group consisting of -H; $NR_{37}R_{38}$, nitro, hydroxyl, halogen, ether, $(C_1-C_8)$alkyl, $(C_1-C_3)$haloalkyl, $(C_2-C_8)$alkenyl, $(C_2-C_3)$haloalkenyl, $(C_3-C_7)$cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl. In another embodiment, optionally in combination with any of the embodiments provided above or below, the $R_3$ functional groups form an electrostatic interaction with $R_1$ groups of the porous polymeric matrix, $R_1$ represents $-R_4N^{(+)}R_5R_6$, being $R_4$ to $R_6$ as defined above, and $R_3$ are negatively-charged functional groups which are the same and are selected from phosphate, sulphate, -COO-, $-SO_{3-}$, -SO-, and $-B(OH)_{2-}$, $R''_{11}$-COO-, $-R''_{12}-SO_{3-}$, $-R''_{13}$-SO-, and $-R''_{14}$- $B(OH)_{2-}$, wherein $R''_{11}$, $R''_{12}$, $R''_{13}$ and $R''_{14}$ are the same or different and are independently selected from the group consisting H, $NR_{37}R_{38}$, nitro, hydroxyl, halogen, ether, $(C_1-C_8)$alkyl, $(C_1-C_8)$haloalkyl, $(C_2-C_8)$alkenyl, $(C_2-C_3)$haloalkenyl, $(C_3-C_7)$ cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl.

In another embodiment, optionally in combination with any of the embodiments provided above or below, the $R_3$ functional groups form an electrostatic interaction with $R_1$ groups of the porous polymeric matrix, $R_3$ functional groups are the same or different and are selected from phosphate, sulphate, -COO-, $-SO_{3-}$, -SO-, and $-B(OH)_{2-}$, $R''_{11}$-$COO_-$, $-R''_{12}-SO_{3-}$, $-R''_{13}$-SO-, and $-R''_{14}-B(OH)_{2-}$, wherein $R''_{11}$, $R''_{12}$, $R''_{13}$ and $R''_{14}$ are the same or different and are independently selected from the group consisting of H, $NR_{37}R_{38}$, nitro, hydroxyl, halogen, ether, $(C_1-C_8)$alkyl, $(C_1-C_8)$haloalkyl, $(C_2-C_8)$alkenyl, $(C_2-C_8)$haloalkenyl, $(C_3-C_7)$ cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl; and $R_1$ represents $-R_4N^{(+)}R_5R_6$, being $R_4$ to $R_6$ the same. In another embodiment, optionally in combination with any of the embodiments provided above or below, the $R_3$ functional groups form an electrostatic interaction with $R_1$ groups of the porous polymeric matrix, $R_3$ functional groups are the same or different and are selected from phosphate, sulphate, -COO-, $-SO_{3-}$, -SO-, and $-B(OH)_{2-}$, $R^{33}{}_{11}$-COO-, -$R^{33}{}_{12}-SO_{3-}$, $-R''_{13}-SO_-$, and $-R''_{14}-B(OH)_{2-}$, wherein $R''_{11}$, $R''_{12}$, $R''_{13}$ and $R''_{14}$ are the same and are independently selected from the group consisting of H, $NR_{37}R_{38}$, nitro, hydroxyl, halogen, ether, (($C_1-C_8$)alkyl, $(C_1-C_8)$haloalkyl, $(C_2-C_8)$alkenyl, $(C_2-C_3)$haloalkenyl, $(C_3-C_7)$ cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl; and $R_1$ represents $-R_4N^{(+)}R_5R_6$, being $R_4$ to $R_6$ the same. In another embodiment, optionally in combination with any of the embodiments provided above or below, the $R_3$ functional groups form an electrostatic interaction with the $R_1$ groups of the porous polymeric matrix, $R_3$ are the same or different and represents a functional group selected from phosphate, sulphate, -COO-, $-SO_{3-}$, -SO-, and $-B(OH)_{2-}$, $R''_{11}$-COO-, $-R^{33}{}_{12}-SO_{3-}$, $-R''_{13}$-SO-, and $-R''_{14}-B(OH)_{2-}$, wherein $R''_{11}$, $R''_{12}$, $R''_{13}$ and $R''_{14}$ are the same or different and are independently selected from the group consisting of H, $NR_{37}R_{38}$, nitro, hydroxyl, halogen, ether, $(C_1-C_8)$alkyl, $(C_1-C_8)$haloalkyl, $(C_2-C_3)$alkenyl, $(C_2-C_3)$haloalkenyl, $(C_3-C_7)$ cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl; and $R_1$ represents $-R_4N^{(+)}R_5R_6$, being $R_4$ to $R_6$ the same and representing a $(C_1-C_{20})$alkyl, $(C_2-C_{20})$alkenyl or $(C_2-C_{20})$alkynyl, particularly $(C_1-C_{20})$alkyl. In another embodiment, optionally in combination with any of the embodiments provided above or below, the $R_3$ functional groups form an electrostatic interaction with the $R_1$ groups of the porous polymeric matrix, $R_3$ are the same and represents a functional group selected from phosphate, sulphate, -COO-, $-SO_{3-}$, $-SO_-$, and $-B(OH)_{2-}$, $R''_{11}$-COO-, $-R''_{12}-SO_{3-}$, $-R''_{13}$-SO-, and $-R''_{14}-B(OH)_{2-}$, wherein $R''_{11}$, $R''_{12}$, $R''_{13}$ and $R''_{14}$ are the same or different and are independently selected from the group consisting of -H and $(C_1-C_{20})$ alkyl optionally substituted with one or more groups selected from H, $NR_{37}R_{38}$, nitro, hydroxyl, halogen, ether, $(C_1-C_8)$alkyl, $(C_1-C_8)$haloalkyl, $(C_2-C_8)$alkenyl, $(C_2-C_3)$haloalkenyl, $(C_3-C_7)$ cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl; and $R_1$ represents $-R_4N^{(+)}R_5R_6$, being $R_4$ to $R_6$ the same and representing a $(C_1-C_{20})$alkyl, $(C_2-C_{20})$alkenyl or $(C_2-C_{20})$alkynyl, particularly $(C_1-C_{20})$alkyl.

Alternatively, in another embodiment of the first aspect of the invention, optionally in combination with any of the embodiments provided above or below, the $R_3$ functional groups form an electrostatic interaction with the $R_1$ functional groups of the porous polymeric matrix, wherein "$R_1$" are negatively charged functional groups and $R_3$ are positively charged functional groups. In another embodiment of the first aspect of the invention, the $R_3$ functional groups form an electrostatic interaction with the $R_1$ functional groups of the porous polymeric matrix, wherein $R_1$ functional groups are the same or different and are selected from -COO-, $-SO_{3-}$, -SO-, and $B(OH)_{2-}$, particularly -COO-; and $R_3$ are positively charged functional groups. In another embodiment of the first aspect of the invention, optionally in combination with any of the embodiments provided above or below, the functional groups $R_3$ form an electrostatic interaction with the $R_1$ functional groups of the porous polymeric matrix, wherein $R_1$ are the same or different and are selected from -COO-, $-SO_{3-}$, -SO-, and $B(OH)_{2-}$, particularly -COO-; and $R_3$ are positively charged functional groups selected from $-R'_4N^{(+)}R'_6R'_5$, and $-P^{(+)}R'_7R'_8R'_9R'_{10}$, wherein $R'_4$ to $R'_{10}$ are the same or different and are independently selected from the group consisting of H, $NR_{37}R_{38}$, nitro, hydroxyl, halogen, ether, $(C_1-C_8)$alkyl, $(C_1-C_8)$haloalkyl, $(C_2-C_8)$alkenyl, $(C_2-C_8)$haloalkenyl, $(C_3-C_7)$ cycloalkyl, and aryl; wherein $R_{37}$ and $R_{38}$ are selected from -H, $(C_1-C_{10})$alkyl, $(C_2-C_{10})$alkenyl, $(C_2-C_{10})$alkynyl, $(C_3-C_7)$ cycloalkyl, and aryl; or, alternatively, $R'_4$, $R'_5$ and $R'_6$ form with the N atom a known saturated or aromatic ring having 5 or 6 members, each one of the members being selected from the group consisting of: -CH-, $-CH_2-$, -NH-, -N-, -SH-, -S-, and -O- (such as imidazoles, pyridines and pyrroles). In another embodiment, optionally in combination with any of the embodiments provided above or below, the $R_3$ functional groups form an electrostatic interaction with the $R_1$ functional groups of

the porous polymeric matrix, and $R_3$ represents $-R'_4N^{(+)}R'_5R'_6$, being $R'_4$ to $R'_6$ as defined above. In another embodiment, the $R_3$ functional groups form an electrostatic interaction with the $R_1$ functional groups of the porous polymeric matrix, $R_3$ represents $-R'_4N^{(+)}R'_5R'_6$, and $R'_4$ to $R'_6$ are the same. In another embodiment, optionally in combination with any of the embodiments provided above or below, the $R_3$ functional groups form an electrostatic interaction with the "$R_1$" groups of the porous polymeric matrix, "$R_3$" represents $-R'_4N^{(+)}R'_5R'_6$, and $R'_4$ to $R'_6$ are the same and represent a $(C_1-C_{20})$alkyl, $(C_2-C_{20})$alkenyl or $(C_2-C_{20})$alkynyl, particularly $(C_1-C_{20})$alkyl. In another embodiment, optionally in combination with any of the embodiments provided above or below, the $R_3$ functional groups form an electrostatic interaction with the $R_1$ functional groups of the porous polymeric matrix, $R_3$ represents $-R'_4N^{(+)}R'_5R'_6$ being $R'_4$ to $R'_6$ as defined above and $R_1$ functional groups are selected from phosphate, sulphate, -COO-, $-SO_{3-}$, -SO-,and $B(OH)_{2-}$, particularly -COO-. In another embodiment, optionally in combination with any of the embodiments provided above or below, the $R_3$ functional groups form an electrostatic interaction with the $R_1$ functional groups of the porous polymeric matrix, $R_1$ functional groups are selected from phosphate, sulphate, COO-, - $SO_{3-}$, -SO-, and $-B(OH)_{2-}$, and $R_3$ represents $-R'_4N^{(+)}R'_5R'_6$, being $R'_4$ to $R'_6$ the same. In another embodiment, optionally in combination with any of the embodiments provided above or below, the functional groups $R_3$ form an electrostatic interaction with the $R_1$ functional groups of the porous polymeric matrix, $R_1$ represents a functional group selected from phosphate, sulphate, COO-, - $SO_{3-}$, -SO-, and $-B(OH)_2$, particularly -COO-, and $R_3$ represents $-R'_4N^{(+)}R'_5R'_6$, being $R'_4$ to $R'_6$ the same and representing a $(C_1-C_{20})$alkyl, $(C_2-C_{20})$alkenyl or $(C_2-C_{20})$ alkynyl, particularly $(C_1-C_{20})$alkyl.

The present invention refers to catalytic systems wherein the catalyst comprises an isoalloxazine moiety of formula (I):

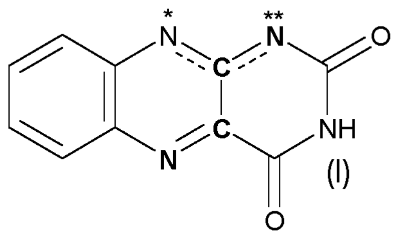

(I)

Its N5 and N1 atoms (bold-highlighted) represent the catalytically reactive positions, because they constitute the conjugated p electron system that can exist in oxidized, one-electron reduced, and two electron reduced forms. The double asterisk at the N1 atom indicates that, when the double bond is located between N10 and C10a, then it has to be bound to a hydrogen. The single asterisk at the N10 atom indicates that, when the double bond is located between N1 and C10a, then it has to be bound to a substituent which takes a particular meaning depending on the particular isoalloxazine. The remaining atoms forming the core can also be substituted, by different groups, depending on the particular isoalloxazine. In one embodiment of the first aspect of the invention, optionally in combination with any of the embodiments provided above or below, the isoalloxazine moiety is of formula (Ibis):

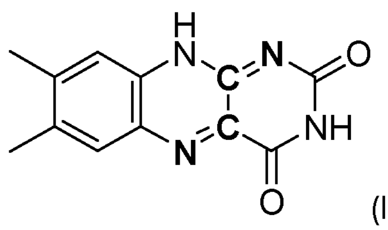

(Ibis)

wherein carbons at positions 6 and 9 as well as the nitrogen at position 10 can be substituted by particular substituents, depending on the particular isoalloxazine.

The isoalloxazine moiety of formula (I) is the main responsible of the redox catalytic activity of the system of the invention. The isoalloxazine group is capable of undergoing oxidation-reduction reactions and can accept either one electron in a two-step process or two electrons at once. Reduction is made with the addition of electrons and hydrogen atoms to specific nitrogen atoms on the isoalloxazine ring system.

The isoalloxazine moiety is often attached with an adenosine diphosphate to form isoalloxazine adenine dinucleotide (FAD), and, in other circumstances, is found as isoalloxazine mononucleotide (or FMN), a phosphorylated form of riboisoalloxazine. It is in one or the other of these forms that isoalloxazine is present as a prosthetic group in flavoproteins. Therefore, flavoproteins are also encompassed by the catalyst forming part of the system of the invention. Illustrative non-limitative examples of isoalloxazine-based catalysts are, therefore, riboisoalloxazine, FMN, FAD, and flavoproteins such as Isoalloxazine oxidases (EC 1.1), Isoalloxazine reductases (EC 1.3), Isoalloxazine monooxygenases (EC 1.1.4) Isoalloxazine decarboxylases (1.2), glutathione reductases (1.8.1), NAD(P)H oxidase (EC 1.6.3) and Isoalloxazine methyl transferases (2.1.1), Isoalloxazine lyases (EC 4.1-2), Isoalloxazine isomerases (EC 5.2) and Isoalloxazine ligases (EC 6.3).

Other natural isoalloxazines and riboisoalloxazine analogues which can form part of the catalytic system of the invention are: riboflavin, lumiflavin, lumichrome, $F_{420}$, 7,8-dimethyl-10-(2,3,4-trihydroxy-4-carboxybutyl)isoalloxazine, 7,8-dimethyl-10-(2,3,4-trihydroxy-4-formylbutyl)isoalloxazine, molybdopterin, 6-hydroxy-7,8-dimethyl-isoalloxazine, 7-methyl-8-hydroxy-isoalloxazine, 8α-hydroxyriboflavin, 7α-hydroxyriboflavin, 8-carboxylumichrome, 7-carboxylumichrome, 8-demethyl-8-dimethylamino-riboflavin, 8-demethyl-8-amino-riboflavin, and 8-demethyl-8-methylamino-riboflavin.

The isoalloxazine rings can already have functional groups falling within the definitions provided above for $R_3$, as it is the case of FMN (which already includes ionizable phosphate groups) or, alternatively, can be chemically modified either to include them or even to control/tune the reversible anchoring of the catalyst to the porous matrix. There is significant literature on the synthesis and functionalization of these "synthetic" isoalloxazines-based catalysts, which also form part of the catalyst system of the invention. For example, the isoalloxazine moiety of formula (I) can be ideally functionalized with ($C_1$-$C_{30}$) aliphatic and aromatic linkers with terminal amino, carboxylic, hydroxyl, thiol, azido, alkynyl groups, iodoacetamide, sulphonates, and maleimide groups, among others, using well-known synthetic protocols (cf. Yoneda F. et al., 1976; Crocker L. et al., 2019; and Pushkareva Z. V. et al., 1966). These chemical modifications can be performed at any position of the molecules of formula (I) except that in the N1 and N5 positions.

The catalyst of the invention can exert the REDOX reaction in response to a stimuli such as light, electromagnetic radiation, heat, magnetic field, difference of potential, or due to the co-immobilization of the catalyst with biological cofactors (NAD+/NADH, glutathione disulfide/glutathione, pyruvate/lactate, dehydroascorbic acid/ascorbic acid, cytochrome C, Fe-S clusters, pyrodoxal phosphate) in the polymeric matrix, or due to a combination thereof.

In one embodiment of the first aspect of the invention, optionally in combination with any of the embodiments provided above or below, the catalytic system further includes a substrate capable of being oxidized or reduced by the isoalloxazine-based catalyst as defined in any of the embodiments provided above. In one embodiment, optionally in combination with any of the embodiments provided above or below, the substrate comprising as reduceable or oxidizable moiety one or more of: C-N bond, a C-O bond, a C-S bond, a C-C bond, a C=N bond, a C=O bond, a C=S bond, a C=N bond, a C=C bond, a C=C bond, a metal or a metal salt.

In one embodiment, optionally in combination with any of the embodiments provided above or below, the substrate is a metal-based substrate. In the present invention the term "substrate" refers to a chemical species upon which a catalyst acts to generate a product, and the term "metal-based substrate" includes metal salts and metal coordination complexes of any nature, including pharmaceutically acceptable salts.

In the present invention, the expression "metal coordination complex" refers to one or more metallic atom(s) or ion(s), called coordination center(s), and a surrounding array of molecules or ions bound to the coordination center(s), that are in turn known as ligands or complexing agents. A coordination complex with a metallic coordination center as metal atom is called a metal complex.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. Examples of pharmaceutical and cosmetical acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, and transition metals. Representative alkali or alkaline earth metal salts include sodium, lithium, potassium, calcium, magnesium, and the like.

In one embodiment, the substrate comprises a noble metal, a transition metal, or any combination or any alloys thereof. Noble metals include silver (Ag), osmium (Os), palladium (Pd), platinum (Pt), gold (Au), rhodium (Rh), ruthenium (Ru), rhenium (Re), iridium (Ir) or any combinations or alloys thereof. Transition metals include iron (Fe), copper (Cu), nickel (Ni), zinc (Zn), cobalt (Co), manganese (Mn), chromium (Cr), molybdenum (Mo), tungsten (W), or tin (Sn), or any combinations or alloys thereof. In another embodiment the metal is a noble metal, in particular Pt. In another embodiment, the substrate is a metal complex of a noble metal, such as Pt, more particularly Pt(IV).

The substrate can be covalently or non-covalently immobilized in the porous polymeric matrix. The particular nature of the immobilization is due, in part, to the physical-chemical properties of the substrate (together with the properties of the system, as explained above).

In one embodiment of the catalytic system of the invention, optionally in combination with any of the embodiments provided above or below, the substrate is non-covalently immobilized to the porous matrix. In another embodiment, optionally in combination with any of the embodiments provided above or below, the substrate is immobilized by electrostatic interactions, particularly ionic interactions, in the porous polymeric matrix. In this embodiment, the substrate is oppositely charged to the porous matrix.

In another embodiment, optionally in combination with any of the embodiments provided above or below, the substrate is immobilized by electrostatic interactions, particularly ionic interactions, in the porous polymeric matrix, and both, the catalyst and the substrate, are negatively charged, whereas the porous polymeric matrix is positively charged. In the demonstration herein presented, the catalyst and the metal-substrate are negatively charged due to phosphate and carboxylate, respectively, whereas the matrix is functionalized with positively charged amine groups (tertiary amines, DEAE). This interaction can also occur "in situ", in the reaction medium, for instance, when the catalytic system of the invention is added to a reaction medium already including the substrate (for example a metal complex) with some charge.

Alternatively, in another embodiment, optionally in combination with any of the embodiments provided above or below, the substrate is immobilized by electrostatic interactions, particularly ionic interactions, in the porous polymeric matrix, and both, the catalyst and the substrate, are positively charged, whereas the porous polymeric matrix is negatively charged.

$R_1$ and $R_2$ are selected to obtain an appropriate immobilization of the catalyst and $R'_1$ to provide an appropriate electronic environment to perform the REDOX reaction.

The technology to covalently couple metal complexes, for example, to polymers is well established and involves the formation, for example of amide bonds, carbamates, triazoles, etc. To form these bonds (cf. W Shen et al., 2015), the metal complexes need to be functionalized with terminal amino groups, carboxylic acids, hydroxyls, alkynes, azides etc. whereas the porous polymeric matrix is functionalized with compatible groups for the immobilization (e.g. amino and carboxylic group, alkyne and azide, etc). The particular conditions and reagents are well-known to those skilled in the art and form part of the common general practice (cf. Greg Hermanson, "Bioconjugate Techniques", Chapter 1, "Introduction to Bioconjugation", Academic Press, 3rd edition, pages 1-125)

Another option that could be considered an "irreversible immobilization" involves the direct coordination of $R_1$ or $R_2$ functional groups to the metal center. Polymers could for example be composed by polyethylene glycol (PEG) functionalized with amines, carboxylic acids, nitriles, pyridines, bipyridines, etc. which are able to coordinate the metal ions constituting the substrate. The particular conditions and reagents are well-known to those skilled in the art and form part of the common general knowledge (cf. Haihua X. et al., 2011; Wen S. et al., 2016).

In one embodiment of the first aspect of the invention, optionally in combination with any of the embodiments provided above or below, the substrate is non-covalently immobilized in the porous polymeric matrix. In another embodiment of the first aspect of the invention, optionally in combination with any of the embodiments provided above or below, the substrate is immobilized in the porous polymeric matrix by electrostatic interactions with $R_1$ or $R_2$ functional groups. In another embodiment of the first aspect of the invention, optionally in combination with any of the embodiments provided above or below, the substrate is immobilized in the porous polymeric matrix by electrostatic interactions with $R_1$ functional groups. In another embodiment of the first aspect of the invention, optionally in combination with any of the embodiments provided above or below, the substrate has negatively charged groups, and it is immobilized in the porous polymeric matrix by electrostatic interactions with $R_1$ positively charged functional groups as defined in any of the embodiments provided above. In another embodiment of the first aspect of the invention, optionally in combination with any of the embodiments provided above or below, the substrate is positively charged, and it is immobilized in the porous polymeric matrix by electrostatic interactions with $R_1$ negatively charged functional groups as defined in any of the embodiments provided above. In another embodiment of the first aspect of the invention, optionally in combination with any of the embodiments provided above or below, the substrate is a metal-based substrate and it is immobilized to the porous polymeric matrix by electrostatic interactions with $R_1$ or $R_2$ functional groups. In another embodiment of the first aspect of the invention, optionally in combination with any of the embodiments provided above or below, the substrate is a metal complex and it is immobilized to the porous polymeric matrix by electrostatic interactions with $R_1$ functional groups as defined in any of the embodiments provided above. In another embodiment of the first aspect of the invention, optionally in combination with any of the embodiments provided above or below, the substrate is a metal complex which has negatively charged groups, and it is immobilized to the porous polymeric matrix by electrostatic interactions with $R_1$ positively charged functional groups as defined in any of the embodiments provided above. In another embodiment of the first aspect of the invention, optionally in combination with any of the embodiments provided above or below, the substrate is a metal complex and it is immobilized to the porous polymeric matrix by electrostatic interactions with either $R_1$ or $R_2$ functional groups as defined in any of the embodiments provided above. In another embodiment of the first aspect of the invention, optionally in combination with any of the embodiments provided above or below, the substrate is a metal

complex and it is immobilized to the polymeric matrix by electrostatic interactions with $R_1$ functional groups as defined in any of the embodiments provided above. In another embodiment of the first aspect of the invention, optionally in combination with any of the embodiments provided above or below, the substrate is a metal complex which has negatively charged groups, and it is immobilized to the polymeric matrix by electrostatic interactions with $R_1$ positively charged functional groups as defined in any of the embodiments provided above. In one embodiment of the first aspect of the invention, optionally in combination with any of the embodiments provided above or below, the substrate has negatively charged groups, which are the same or the different, and are selected from: phosphate, sulphate, carboxylate, succinate, methylsuccinate, glutarate, maleate, and phthalate, and $R_1$ groups are $-R_4N^{(+)}R_5R_6$, being $R_4$ to $R_6$ as defined in any of the embodiments provided above. In another embodiment of the first aspect of the invention, optionally in combination with any of the embodiments provided above or below, the substrate has negatively charged succinate groups, and $R_1$ groups are $-R_4N^{(+)}R_5R_6$, being $R_4$ to $R_6$ the same or different and representing $(C_1-C_{20})$alkyl.

The loading of substrate within the catalytic system depends on the intended industrial application and can be tuned on the basis of the porosity degree (as explained above) and the amount of $R_1$ or $R_2$ functional groups. The skilled person, using their general knowledge, can routinely determine the appropriate loading and how to adapt the porosity and the $R_1$ or $R_2$ concentration. In one embodiment of the first aspect of the invention, optionally in combination with any of the embodiments provided above or below, the $R_1$ or $R_2$ functional groups are at a molar concentration excess with respect to the molar concentration of the substrate within the catalytic system (expressing the concentration of the substrate and the $R_1$ functional groups with the same units such as $\mu$mol/gram of matrix).

In the present invention, the term "molar concentration" when referred to $R_1$, $R'_1$, $R_2$, $R_3$, the substrate or catalyst, refers to the number of moles of the particular component per gram of matrix. When reference is made in the present invention to the fact that a particular component (for example $R_1$ functional groups) is in a molar concentration excess with respect to the catalyst, it means that the number of moles of that component, per gram of matrix, is higher than the number of moles of catalyst. When reference is made in the present invention to the fact that the catalyst is in molar concentration defect with respect to $R_1$ or $R_2$, it means that the number of moles of catalyst, per gram of matrix, is lower than the number of moles of $R_1$ or $R_2$.

In another embodiment of the first aspect of the invention, the substrate is at a molar concentration ratio, with respect to the molar concentration of $R_1$ or $R_2$ groups, comprised from 1:1.5 to 1:50, from 1:8 to 1:25 or from 1:10 to 1:20.

In another embodiment of the first aspect of the invention, the substrate is at a molar concentration excess with respect to the molar concentration of catalyst. In another embodiment of the first aspect of the invention, the substrate is at a molar concentration ratio, with respect to the molar concentration of the catalyst, comprised from 1.5:1 to 50:1, from 8:1 to 25:1 or from 10:1 to 20:1.

$R_1$ or $R_2$ functional groups in the porous polymeric matrix, which are the functional groups responsible for anchoring both the catalyst and the substrate, are in a molar concentration excess with respect to the total concentration of both, the catalyst and the substrate. In another embodiment of the first aspect of the invention the ratio between the molar concentration of $R_1$ groups and the total concentration of catalyst and substrate is at least of 5:1 or at least of 10:1. In another embodiment of the first aspect of the invention the ratio between the molar concentration of $R_1$ groups and the total concentration of catalyst and substrate is comprised from 5:1 to 50:1, from 10:1 to 30:1 or from 12:1 to 18:1.

In another embodiment, $R'_1$ functional groups are at a molar concentration excess with respect to the molar concentration of substrate. In another embodiment of the first aspect of the invention the ratio between the molar concentration of $R'_1$ groups and the molar concentration of catalyst and/or substrate is at least of 50:1, at least of 100:1 or at least of 150:1. In another embodiment of the first aspect of the invention, the ratio between the molar concentration of "$R_1$" groups and the substrate is comprised from 50:1 to 300:1. In another embodiment of the first aspect of the invention the ratio between the molar concentration of "$R_1$" groups and the substrate is 200:1.

In another embodiment, the substrate is at a molar concentration excess with respect to the molar concentration of catalyst. In another embodiment, the substrate is at a molar concentration ratio, with respect to the molar concentration of the catalyst, comprised from 1.5:1 to 50:1, from 8:1 to 25:1 or from 10:1 to 20:1.

In another embodiment of the first aspect of the invention, optionally in combination with any of the embodiments provided above or below, the catalytic system further includes one or more REDOX cofactor(s), such as NADH/NAD+, glutathione disulfide/glutathione, pyruvate/lactate, dehydroascorbic acid/ascorbic acid, cytochrome C, Fe-S clusters and pyridoxal phosphate. These co-factors can also be reversibly or irreversibly immobilized in the porous polymeric matrix by routine means. In one embodiment, these cofactors can be anchored reversibly via non-covalent interactions, prevalently electrostatic (as described above for the catalyst), with the $R_1$ or $R_2$ groups of the polymer and the polar, ionizable groups present in their structure.

In another embodiment of the first aspect of the invention, optionally in combination with any of the embodiments provided above, the catalytic system comprises (a) an optionally cross-linked porous polymeric matrix made of saccharide monomers, such as agarose, optionally cross-linked, the matrix being functionalized with positively charged $R_1$ groups, and (b) an isoalloxazine-based catalyst with negatively charged $R_3$ groups, wherein the porous polymeric matrix has one or more of the following features: (i) it further comprises a substrate, which is a metal-based substrate such as a metal salt

or complex, which is electrostatically immobilized to the porous polymeric matrix (by interactions with the $R_1$ functional groups), (ii) the $R_1$ functional groups are at a molar concentration excess with respect to the molar concentration of catalyst, particularly the molar concentration ratio of $R_1$ functional groups vs molar concentration of catalyst is of at least 50, particularly of at least 100, of at least 150, particularly of 200; (iii) if present, the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20); and (iv) the $R_1$ are electron donor groups, such as $-R_4N^{(+)}R_5R_6$, being $R_4$ to $R_6$ as defined above. In another embodiment of the first aspect of the invention, optionally in combination with any of the embodiments provided above, the catalytic system comprises (a) a porous polymeric matrix made of saccharide monomers, such as agarose, optionally cross-linked, the porous polymeric matrix having a porosity degree of at least 50%, 60%, 70%, 80% or 90% and pores with a size from 40 to 600 nm, from 50 to 400 nm, from 60 to 300 nm or from 70 to 200 nm, the matrix being functionalized with positively charged $R_1$ groups, and (b) an isoalloxazine-based catalyst with negatively charged $R_3$ groups, wherein the porous polymeric matrix has one or more of the following features: (i) it further includes a substrate, which is a metal-based substrate such as a metal salt or complex, which is electrostatically immobilized to the porous polymeric matrix (by interactions with the $R_1$ functional groups), (ii) the $R_1$ functional groups are at a molar concentration excess with respect to the molar concentration of catalyst, particularly the molar concentration ratio of $R_1$ functional groups vs molar concentration of catalyst is of at least 50, particularly of at least 100, of at least 150, particularly of 200; (iii) if present, the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20); and (iv) the $R_1$ are electron donor groups, such as $-R_4N^{(+)}R_5R_6$, being $R_4$ to $R_6$ as defined above.

In another embodiment of the first aspect of the invention, optionally in combination with any of the embodiments provided above, the catalytic system comprises (a) a porous polymeric matrix made of saccharide monomers, such as agarose, optionally cross-linked, the matrix being functionalized with positively charged $R_1$ groups, (b) an isoalloxazine-based catalyst with negatively charged $R_3$ groups, and (c) a substrate, which is a metal-based substrate such as a metal salt or complex, particularly a metal prodrug, which is electrostatically immobilized to the porous polymeric matrix (by interactions with the $R_1$ functional groups), wherein (i) the $R_1$ functional groups are at a molar concentration excess with respect to the catalyst, particularly the molar concentration ratio of $R_1$ functional groups vs molar concentration of catalyst is of at least 50, particularly of at least 100, of at least 150, particularly of 200; (ii) the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect to $R_1$ functional groups (particularly at a molar concentration ratio, with respect to the molar concentration of $R_1$ groups, comprised from 1:1.5 to 1:50, from 1:8 to 1:25 or from 1:10 to 1:20); and (iii) the $R_1$ are electron donor groups, such as $-R_4N^{(+)}R_5R_6$, being $R_4$ to $R_6$ as defined above, particularly being the same, particularly being a $(C_1-C_{20})$alkyl. In another embodiment of the first aspect of the invention, optionally in combination with any of the embodiments provided above, the catalytic system comprises (a) a porous polymeric matrix made of saccharide monomers, such as agarose, optionally cross-linked the porous polymeric matrix having a porosity degree of at least 90% and pores with a size from 40 to 600 nm, from 50 to 400 nm, from 60 to 300 nm or from 70 to 200 nm, the matrix being functionalized with positively charged "$R_1$" groups, (b) an isoalloxazine-based catalyst with negatively charged $R_3$ groups, and (c) a substrate, which is a metal-based substrate such as a metal salt or complex, particularly a metal prodrug, which is electrostatically immobilized to the porous polymeric matrix (by interactions with the $R_1$ functional groups), wherein (i) the $R_1$ functional groups are at a molar concentration excess with respect to the catalyst, particularly the molar concentration ratio of $R_1$ functional groups vs molar concentration of catalyst is of at least 50, particularly of at least 100, of at least 150, particularly of 200; (ii) the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20);, and (iii) the $R_1$ are electron donor groups, such as $-R_4N^{(+)}R_5R_6$, being $R_4$ to $R_6$ as defined above, particularly being the same, particularly being $(C_1-C_{20})$alkyl groups.

In another embodiment of the first aspect of the invention, optionally in combination with any of the embodiments provided above, the catalytic system comprises (a) a porous polymeric matrix made of saccharide monomers, such as agarose, optionally cross-linked, the matrix being functionalized with positively charged $R_1$ groups, (b) an isoalloxazine-based catalyst, particularly FMN, with negatively charged $R_3$ groups (i.e., the phosphate groups for the case of FMN), and (c) a substrate, which is a metal complex prodrug which is electrostatically immobilized to porous polymeric matrix (by interactions with the $R_1$ functional groups), wherein (i) the $R_1$ functional groups are at a molar concentration excess with respect to the catalyst, particularly the molar concentration ratio of $R_1$ functional groups vs molar concentration of catalyst is of at least 50, of at least 100, of at least 150, particularly of 200; (iii) the substrate is at a molar concentration excess with

respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20); and (iii) the $R_1$ are electron donor groups, such as $-R_4N^{(+)}R_5R_6$, being $R_4$ to $R_6$ as defined above, particularly being the same, particularly being a $(C_1-C_{20})$alkyl. In another embodiment of the first aspect of the invention, optionally in combination with any of the embodiments provided above, the catalytic system comprises (a) a porous polymeric matrix made of saccharide monomers, such as agarose, optionally cross-linked, the porous polymeric matrix having a porosity degree of at least 90% and pores with a size from 40 to 600 nm, from 50 to 400 nm, from 60 to 300 nm or from 70 to 200 nm, the matrix being functionalized with positively charged $R_1$ groups, (b) an isoalloxazine-based catalyst, particularly FMN, with negatively charged $R_3$ groups (i.e., the phosphate groups for the case of FMN), and (c) a substrate, which is a metal complex, which is electrostatically immobilized to the porous polymeric matrix (by interactions with the $R_3$ functional groups, i.e., the phosphate groups in the case of FMN), wherein (i) the $R_1$ functional groups are at a molar concentration excess with respect to the catalyst, particularly the molar concentration ratio of $R_1$ functional groups vs molar concentration of catalyst is of at least 50, particularly of at least 100, of at least 150, particularly of 200; (ii) if present, the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20); and (iii) the $R_1$ are electron donor groups, such as $-R_4N^{(+)}R_5R_6$, being $R_4$ to $R_6$ as defined above, particularly being the same, particularly being $(C_1-C_{20})$alkyl groups.

In another embodiment of the first aspect of the invention, optionally in combination with any of the embodiments provided above, the catalytic system comprises (a) a porous polymeric matrix made of saccharide monomers, such as agarose, optionally cross-linked, the matrix being functionalized with positively charged $R_1$ groups, (b) an isoalloxazine-based catalyst, particularly FMN, with negatively charged $R_3$ groups (i.e., the phosphate groups for the case of FMN), and (c) a substrate, which is a Pt(IV) complex prodrug which is electrostatically immobilized to porous polymeric matrix (by interactions with the $R_3$ functional groups,), wherein (i) the $R_1$ functional groups are at a molar concentration excess with respect to the catalyst, particularly the molar concentration ratio of $R_1$ functional groups vs molar concentration of catalyst is of at least 50, particularly of at least 100, of at least 150, particularly of 200; (ii) the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20); and (iii) the $R_1$ are electron donor groups, such as $-R_4N^{(+)}R_5R_6$, being $R_4$ to $R_6$ as defined above, particularly being the same, particularly being a $(C_1-C_{20})$alkyl. In another embodiment of the first aspect of the invention, optionally in combination with any of the embodiments provided above, the catalytic system comprises (a) a porous polymeric matrix made of saccharide monomers, such as agarose, optionally cross-linked, the porous polymeric matrix having a porosity degree of at least 90% and pores with a size from 40 to 600 nm, from 50 to 400 nm, from 60 to 300 nm or from 70 to 200 nm, the matrix being functionalized with positively charged "$R_1$" groups, (b) an isoalloxazine-based catalyst, particularly FMN, with negatively charged $R_3$ groups (i.e., the phosphate groups for the case of FMN), and (c) a substrate, which is a Pt(IV) complex prodrug which is electrostatically immobilized to the porous polymeric matrix (by interactions with the $R_3$ functional groups, i.e., the phosphate groups in the case of FMN), wherein (i) the $R_1$ functional groups are at a molar concentration excess with respect to the catalyst, particularly the molar concentration ratio of $R_1$ functional groups vs molar concentration of catalyst is of at least 50, particularly of at least 100, of at least 150, particularly of 200; (ii) the substrate is at a molar concentration excess with respect to the catalyst (particularly at a molar concentration ratio, with respect to the molar concentration of the catalyst, comprised from 1.5:1 to 50:1, from 8:1 to 25:1 or from 10:1 to 20:1), and in defect with respect $R_1$ functional groups (particularly at a molar concentration ratio, with respect to the molar concentration of $R_1$ groups, comprised from 1:1.5 to 1:50, from 1:8 to 1:25 or from 1:10 to 1:20);, and (iii) the $R_1$ are electron donor groups, such as $-R_4N^{(+)}R_5R_6$, being $R_4$ to $R_6$ as defined above, particularly being the same, particularly being $(C_1-C_{20})$alkyl groups.

Thus, in another embodiment of the first aspect of the invention, the catalytic system is a particulate catalytic system comprising a plurality of particles each of them comprising the features of the invention and with a particle size between 1 micrometer and 1 cm.

And yet in a final embodiment of the first aspect of the invention, the catalytic system is a continuous catalytic system forming a continuous surface where the longest dimension is between 1 mm and 100 meters.

In a second aspect the present invention provides a process for preparing a catalytic system as defined in the first aspect, the process comprising the mixing of the porous polymeric matrix and the catalyst, wherein the catalyst is added at a molar concentration in defect with respect to the molar concentration of $R_1$ or $R_2$ functional groups included in the polymeric matrix, under appropriate conditions.

In the present invention the term "under appropriate conditions" when referred to the process of the second

aspect of the invention, means those conditions that allow the formation of the non-covalent reversible interactions between the catalyst and the anchoring functional groups of the polymeric matrix.

In one embodiment of the second aspect, optionally in combination with any of the embodiments provided above or below, the process further includes the step of adding a substrate as defined in any of the embodiments of the first aspect of the invention. In another embodiment, optionally in combination with any of the embodiments provided above or below, the process is performed by sequentially adding first the substrate and then the catalyst to the porous matrix. In one embodiment of the second aspect of the invention, optionally in combination with any of the embodiments provided above or below, the substrate is added at a molar concentration in defect with respect to the molar concentration of $R_1$ or $R'_1$. In another embodiment of the second aspect of the invention, optionally in combination with any of the embodiments provided above or below, the process further comprises the step of adding one or more REDOX co-factors which can be selected from NADH/NAD+, glutathione disulfide/glutathione, pyruvate/lactate, dehydroascorbic acid/ascorbic acid, cytochrome C, Fe-S clusters and pyridoxal phosphate, particularly NADH/NAD+. In another embodiment of the second aspect of the invention, optionally in combination with any of the embodiments provided above or below, the process is performed by sequentially adding first the substrate, then the catalyst and last the REDOX co-factor to the porous matrix.

All the embodiments provided above under the first aspect of the invention, regarding the porous matrix, catalyst and substrate, are also embodiments of the second aspect of the invention.

In one embodiment of the second aspect, when the non-covalent reversible interaction between the catalyst and the porous matrix is of electrostatic nature, the process comprises the step of adding the catalyst to the porous matrix at appropriate conditions of ionic strength and pH which favors the ionization of the ionizable functional groups $R_1$ (or $R_2$) and $R_3$, thus promoting the generation of the electrostatic interaction. In another embodiment of the process for obtaining this catalytic system based on electrostatic interactions, the process further comprises the addition of a substrate. In another embodiment of the process for obtaining this catalytic system based on electrostatic interactions, the process is performed by sequentially adding firstly the substrate and then the catalyst to the porous matrix. In this embodiment, the appropriate conditions of ionic strength and pH promote not only the ionization of the porous matrix's functional $R_1$ groups, but also the generation of an opposite charge in the substrate and catalyst thus promoting their electrostatic interaction with the $R_1$ or $R_2$ functional groups of the matrix. In another embodiment of the process for obtaining this catalytic system based on electrostatic interactions, the process can further include optional steps, such as the addition of one or more co-factors, for instance, as defined above. In another embodiment of the process for obtaining a catalytic system based on electrostatic interactions the process comprises the step of adding the substrate and then the catalyst to a porous matrix, the pH of the medium being adjusted from 7 to 9. In another embodiment of the second aspect, the present invention provides a process for obtaining a catalytic system based on electrostatic interactions between the porous polymeric matrix, which is functionalized with positively charged groups of formula $-R_4N^{(+)}R_6R_5$ or $-P^{(+)}R_7R_8R_9R_{10}$, wherein $R_4$ to $R_{10}$ are as defined above, and the catalyst, which is functionalized with negatively charged $R_3$ groups, such as phosphate groups, the process comprising the step of adding the substrate and then the catalyst to a porous matrix, the pH from 7 to 9 and the ionic strength being adjusted by adding a buffer such as Tris-HCl 10 mM.

In one embodiment of the second aspect, the invention provides a process for preparing a catalytic system wherein the catalyst interacts with $R_1$ or $R_2$ functional groups by hydrogen bonding. In this embodiment, the process comprises the step of adding the catalyst to the porous matrix under appropriate conditions of ionic strength and pH in which it is promoted the generation of hydrogen bonding interaction between an hydrogen atom covalently bound to an electronegative atom forming part of anchoring group ($R_1$ or $R_2$), and an electronegative atom forming part of the $R_3$ functional group or vice versa (i.e., between an hydrogen atom covalently bound to an electronegative atom forming part of $R_3$, and an electronegative atom forming part of the anchoring functional group ($R_1$ or $R_2$)). Alternatively, the porous matrix is suspended in a suitable organic solvent and the catalyst added at the conditions in which the functional groups $R_1$ (or $R_2$) and $R_3$ are in their neutral form, thus promoting the generation of hydrogen bonding interaction.

In one embodiment of the second aspect, the invention provides a process for preparing a catalytic system wherein hydrophobic $R_3$ functional groups of the catalyst reversible interact with hydrophobic $R_1$ or $R_2$ functional groups, particularly with hydrophobic $R_2$ functional groups, by hydrophobic interaction. In this embodiment, the porous matrix is a particulate porous matrix and is suspended in a mixture of (a) water and (b) a non-water soluble organic solvent wherein the catalyst, functionalized with hydrophobic $R_3$ functional group, is soluble. The catalyst bearing hydrophobic $R_3$ groups is added to the reaction medium comprising the matrix suspended in the mixture, and the catalyst is allowed to slowly diffuse inside the pores of the matrix, wherein it will be retained by interacting, through its hydrophobic $R_3$ functional groups, with the hydrophobic $R_1$ or $R_2$ functional groups of the porous matrix. If required, the porous matrix is collected by centrifugation and finally washed with a mixed water/organic solvent system.

As it is well-known for the skilled person in the art, a parameter useful to determine whether a molecule is hydrophilic or hydrophobic is determining its partition coefficient (P), which has been defined above.

In a third aspect the present invention provides a catalytic system as defined in the first aspect of the invention for use in therapy or diagnostics.

The type of substrate immobilized in the porous polymeric matrix will determine the particular use of the system.

Thus, if the substrate is a metal-based prodrug, for instance, the system is useful in therapy.

If the substrate included in the catalytic system of the invention is a metal complex whose luminescence is switched on by a REDOX reaction then the system is useful for detecting metal ions in a specific oxidation state. For example, a metal ion to be detected, e.g. Cu(II), should be incubated in the medium outside the matrix with a suitable coordinating ligand that gives a non-luminescent complex. This can diffuse and enter the matrix where it undergoes reduction to form a Cu(I) analogue that is fluorescent and allows detection (inside or outside the polymer matrix).

In a further aspect the present invention provides a pharmaceutical or cosmetical composition comprising the catalytic system as defined above, together with one or more therapeutic or cosmetical excipients and/or carriers. All the embodiments provided under the first aspect of the invention are also embodiments of the composition of the invention. In one embodiment the composition is a topical composition. In another embodiment, the composition is a topical composition and the catalytic system as defined in the first aspect of the invention includes a metal-based substrate prodrug in a therapeutically effective amount.

The expression "therapeutically effective amount" as used herein, refers to the amount of a compound (here the product resulting from the redox reaction) that, when administered, is sufficient to prevent development of, or alleviate to some extent, one or more of the symptoms of the disease which is addressed. The particular dose of the metal-based substrate to be included in the catalytic system will be determined by the particular circumstances surrounding the case, including the particular compound to be administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The formulations of the pharmaceutical compositions described herein may be prepared by any method known or hereafter developed in the art of pharmacology. In general, such preparatory methods include the step of bringing the active ingredient (the catalytic system) into association with an excipient and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

The composition of the third aspect of the invention can also be useful in cosmetics. The expression "cosmetically effective amount" as used herein, refers to the amount of a compound (here the product resulting from the redox reaction) that, when administered, is sufficient to provide the desired cosmetic effect.

In one embodiment of this particular cosmetic application, the catalytic system of the invention can be used to synthesize fragrances from their corresponding precursors, the resulting fragrances being released over time, for example under sun light.

Alternatively, a sun-screen oxidizable/reducible substrate can also be formulated within the catalytic system of the invention. Thus, for example, the substrate could be ferulic acid, which would be formulated within the catalytic system (consisting of, for example, agarose microbeads functionalized with positively charged $R_1$ groups, particularly functionalized with diethylaminoethyl groups that act as electron donors but also as anchoring groups for the catalyst (FMN)). With sun light, the catalytic reaction would start, producing eugenol which would be released from the matrix due to the loss of its carboxylic group. This system can be easily applied as cream to the screen, which after sunlight irradiation the system will autonomously release the fragrance.

In a further aspect the present invention provides a device comprising the catalytic system of the invention, such as a medical device, more particularly an implantable medical device.

The devices of the invention can be manufactured by routine techniques commonly used in this particular field. The technique will depend on the final manufacture product.

For instance, if the medical device is an implantable wafer, mesh or stent, the device can be manufactured

applying a coating of the catalytic system already including the prodrug substrate (for example by dipping). The medical device thus obtained can be implanted in the host and just applying a mild external stimuli (for example light) can start the redox reaction of the prodrug and the subsequent delivery of the active drug species through the pores to reach the target site (i.e. the part of the body to be treated). In this embodiment, the porous polymeric matrix has to be selected among those well-recognized as appropriate to be in contact with animals. In one embodiment, the implantable medical device is a biodegradable implantable medical device. In this embodiment the porous polymeric matrix is a biodegradable porous polymeric matrix (cf. Wolinsky J. B. et al., 2012). In this embodiment the stent is implanted close to the part of the body to be treated.

Another example of device according to the invention is the manufacture of a patch. With this device, the catalytic composition already including the prodrug substrate can be topically applied. The patch can also be obtained by routine protocols, such as by applying a coating of the catalytic system to a carrier material. The term "carrier material" has to be understood as a substrate of suitable material allowing depositing the catalytic system thereon, its transport and its release at the desired site, for example, in the site where it has to exert its therapeutic effect. Said carrier material can be a solid support, such as dressings, band-aids, compresses, plasters, etc. In one embodiment, the material carrier is a solid support made of collagen, gelatin or oxidized regenerated cellulose.

Another example of device is a reactor comprising the catalytic system of the invention. The reactor can find application in the treatment of liquid effluents where a REDOX catalysis is sought, such as treatment of residual waters, solutions proceeding from mining processes such as hydrometallurgy, bioremediation and fabrication of chemical structures. When the device is used as a reactor, the substrate is usually present in the liquid effluent to be treated and has to diffuse, through the pores of the polymeric matrix, inside the catalytic system. The manufacture of the reactor of the invention, for instance, can be performed by introducing the particulated catalytic system of the invention within a container comprising inlet and outlet ports to allow the admission of the liquid effluent containing the substrate and the extraction of the product of the catalysed reaction. Means such as filters can be implemented in the liquid transfer ports to prevent the escape of the particulated catalytic system through the inlet and outlet ports. Alternatively, solid elements integral to the reactor and located in the inner volume thereof such as the inner walls or other elements placed within the reactor for this purpose, can be coated with a continuous layer of the catalytic system of the invention, thereby allowing for the treatment of the liquid effluent containing the substrate and eliminating the need to separate the product from the catalytic system. Different geometrical designs and operation modes can accommodate various processing modes, for example, batch processing can be performed employing cubic or cylindrical reactors comprising a stirred for increasing the access of substrate molecules to the catalytic system. Alternatively, pipes of various cross-section geometries can be used as reactors in flow-mode systems. The person skilled in the art can find several other geometries and operation modes that can be accommodated by the general description exemplified herein. Alternatively, the reactor can be constituted catalytic systems suspended in suitable chamber under stirring and external stimuli (e.g. light or REDOX co-factor). The catalytic systems can be operated both in continuous and discontinuous modes through its integration to packed-bed and batch reactors, respectively.

In a final aspect the present invention provides an ex *vivo* method for performing a REDOX catalytic reaction of a substrate capable of being reduced or oxidized by a catalyst comprising an isoalloxazine moiety, the method comprising (a) contacting the substrate with the catalytic system as defined above, and (b) applying an external stimuli selected from light, temperature, a REDOX cofactor, differential voltage applied between electrodes, and a combination thereof.

All the embodiments provided above regarding the substrate, co-factors, etc., are also embodiments of the ex vivo method of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

## Examples

Methods

UV-Vis measurements were carried out with a plate reader Biotek EPOCH using monochromators. The concentration of FMN was measured by monitoring its absorbance at 450 nm in the supernatant of the immobilization experiments.

NMR spectra were recorded on an AVANCE III Bruker 500 NMR using standard pulse programs. Chemical shifts

were reported in parts-per-million (δ, ppm) and referenced to the residual solvent peak.

Liquid chromatography was performed on an Acquity UPLC system using an Acquity BEH C18 column (100x 2.1 mm, 1.7 μm) from Waters (Milford, MA, USA) and equipped with photodiode array detector (PDA). The gradient elution buffers were A (1% TFA in water) and B (1% TFA in acetonitrile). The gradient method was: 0-1 minutes, isocratic at 100% A; 1-10 minutes, gradient to 20% A; 10-11 minutes, gradient to 100% A; 11-14 minutes of stabilization at 95% A. The UV detector wavelength was set at 254 nm and the injection volume was 10 μL. Total run time was 14 minutes while the flow rate was set at 300 $\mu L \cdot min^{-1}$.

The mass spectrometry detection was carried out using a time-of-flight mass spectrometer (ESI-TOF). LCT Premier XE from Waters (Milford, MA, USA) with an electrospray ionization source, working in positive/V mode. The MS range acquired was between m/z 50-1,000. The capillary and cone voltages were set at 3,000 and 50 V, respectively. Desolvation gas temperature was 300°C and source temperature was 120°C. The desolvation gas flow was set at 600 $L \cdot h^{-1}$ and cone gas flow was set at 50 $L \cdot h^{-1}$.

For quantification and data analysis, Masslynx v4.1 software was used (Waters, Milford, MA, USA). All the analytes were identified by mass spectrometry.

Scanning Electron Microscope and Energy Dispersive X-ray (SEM-EDX) images were analyzed with a SEM-Quanta FEG-250, ESEM microscope employing a large field detector (LFD). For imaging, 5 μL water suspension (1:20) of each sample were deposited in the top of carbon conductive tabs. Images were acquired at 10 keV under 150 Pa.

XPS experiments were performed in a SPECS Sage HR 100 spectrometer with a non-monochromatic X-ray source (Magnesium Kα line of 1253.6 eV energy and 252 W), placed perpendicular to the analyzer axis and calibrated using the 3d5/2 line of Ag with a full width at half maximum (FWHM) of 1.1 eV. The selected resolution for the spectra was 15 eV of Pass Energy and 0.15 eV/step. All measurements were made in an ultra-high vacuum (UHV) chamber at a pressure around $8 \cdot 10^8$ mbar. An electron flood gun was used to neutralize for charging. C 1s sp3 from adventitious carbon was used for charge-correcting the spectra and fixed at 284.8 eV. Peak assignation was done according to JF Moulder and NIST database.

Single-particle fluorescence microscopy. 184 μL of 1:200 (w/v) suspension of 1-FMN@AGM-D in 10 mM Tris-HCl buffer at pH 7.6 were placed into a well in an 8-well μslide. The autofluorescence of FMN ($\lambda_{ex}$: 470 nm, $\lambda_{em}$: 500-557 nm) within each microbead was collected by using a Cell Axio Observer microscope with the Colibri LED illumination system incorporated. Images were then analyzed by using the software ImageJ.

## Example 1: FMN-based catalytic system

### 1.1. Preparation of the catalytic system

In this example, the isoalloxazine-based catalyst used was FMN [Merck], and the porous polymeric matrix consisted of agarose microbeads (AGM) [ABT technologies (6% cross-linked Agarose Bead Standard], functionalized with diethylaminoethyl groups (AGM-D). The microbeads of agarose, according to manufacturer's instructions have an average particle size of 90 μm with an average pores size of 70-200 nm measured by SEM.

For the case of the agarose wet matrix, ρ = is 1.64 $g \times mL^{-1}$ according to Laurent et al. (cf. Laurent T. C. et al., 1967) and ω = 0.625 according to Jonhson et al. (cf. Johnson E. M. et al., 1995). For 6% cross-linked agarose (0.06 $g \times mL^{-1}$), ϕ = 0.0585. Therefore, the void volume (porosity degree) of the agarose bead is (1-100 × ϕ) = 94,15 %. The density of agarose was measured using a 1 mL cylinder and filling it with wet agarose at 25°C and atmospheric pressure. Then, the cylinder with the agarose was weighted and subtracted the weight of the empty cylinder.

The AGM were functionalized with positively-charged diethylaminoethyl groups (AGM-D) by firstly modifying AGM with epoxy groups using epichlorohydrine [Merck] in basic media (pH 11-13) during 16 hours at 25°C under vigorous stirring using a paddle stirrer at 100 rpm. After the epoxidation step, the AGM was washed with an excess of distilled water and finally incubated with a solution of 1M of 2,2'-diethyl aminoethanol (DEAE) in a ratio 1:10 (w:v), during 16 hours at 25°C. The resulting AGM-D were washed with an excess of water and the functionalized polymeric matrix was vacuum filtered and stored at 4°C. The confirmation that the resulting AGM was functionalized with DEAE groups was made by X-ray photoelectron spectroscopy (XPS): peaks corresponding to C-N bonds forming the secondary (398 eV) and tertiary amines (402 eV) from DEAE (i.e. C-N bonds between the porous matrix and the nitrogen atom of the DEAE) were detected.

To quantify the concentration of DEAE groups, 0.1 g of the matrix before and after the functionalization was incubated with 1mL of 1 M of $H_2SO_4$ for 1 hour, and then rinsed with 10 mL of distilled water and vacuum filtered. In this step the epoxide groups were transformed into diols groups that were further oxidized under mild conditions. 0.1 g of each matrix after the hydrolysis step was incubated with 1 mL of 10 mM of sodium metaperiodate for 2 hours, and the non-reactive periodate of the supernatant of that suspension was titrated with a solution of 10% of potassium iodide in a bicarbonate saturated solution. According to the concentration of consumed sodium metaperiodate, it was calculated the number of the diols resulting from the epoxide hydrolysis, and consequently the concentration of epoxy groups. The difference of the epoxy groups in the matrix before and after the incubation with DEAE means the amount of DEAE groups

on the agarose matrix. Following this protocol, it was found that there was a concentration of DEAE of about 100 μmol × $g^{-1}$

Then, 1 mL of a 75 μM FMN solution (from Merck Ref: F2253) were incubated with 100 mg AGM-D for 10 minutes at 25° C, under the low ionic strength and neutral pH conditions provide by 10 mM Tris-HCl (pH 7.6). The suspension was gently stirred with a wheel shaker (50 rpm).

Subsequently, 1 mL of a 1 mM aqueous solution of the complex cis,cis,trans-$[Pt(NH_3)_2(Cl_2)(O_2CCH_2CH_2CO_2H_2)_2]$ (1) (prepared as disclosed by Reithofer M. and colleagues (Reithofer M. et al., 2006)) was incubated with the 100 mg AGM-D already loaded with FMN under the same immobilization conditions previously described (10 minutes at 25°C at pH 7.5 in 10 mM Tris-HCl buffer). The suspension was gently stirred with a wheel shaker (50 rpm).

On the one hand, it was confirmed by scanning electron microscopy/electron diffraction X-ray (SEM/EDX) (Fig.1) the presence of Pt(IV) complex inside the beads.

Single-particle fluorescence microscopy was employed to localize the flavin catalyst with respect to the polymer matrix at a single-particle level. Upon excitation of the catalytic system 1-FMN@AGM-D at 470 nm (prepared as previously described), the green fluorescence emission of FMN at 520 nm confirmed that the immobilization of the flavin catalyst was homogeneous within the whole polymer matrix.

On the other hand, it was also confirmed by UV-Vis (representative peak for FMN at 450 nm and for Pt(IV)-complex at 220 nm) that the loading of flavin catalyst and metal prodrug into AGM-D were 0.5 and 6 μmol· $g^{-1}$, respectively. The amount of metal prodrug within the catalytic system corresponded to therapeutical doses. The loading of each molecule was determined by UV-Vis according to a standard calibration curve. The absorbance at the representative peak of each molecule was measured for a calibration curve of samples with known concentrations. The calibration curve was adjusted to a linear regression with the formula $y = a + bx$, where y is the measured absorbance of each sample at the representative peak and x is the known concentration of each sample. The catalytic system resulting from the above protocol, including the Pt(IV) compex, FMN, and AGM-D is referred hereinafter as 1-FMN@AGM-D.

The molar ratio between the Pt(IV) complex and the FMN was calculated by dividing the concentration of each molecule immobilized on AGM-D matrix (μmol(PtIV-complex) × g(AGM-D)$^{-1}$ / μmol(FMN) × g(AGM-D)$^{-1}$). Finally, the catalytic system was then washed one time with 10 mM Tris-HCl (pH 7.6) in a ratio 1:10 (w:v) for 5 minutes. The washing suspension was gently stirred with a wheel shaker (50 rpm).

In this catalytic system the final concentration of each one of the components was: 100 μmol DEAE, 0.5 μmol of FMN and 6 μmol Pt(IV) complex all of them per gram of agarose matrix. This means that DEAE groups were at a molar excess with respect to the catalyst (200-fold higher) and with respect to the substrate, the Pt(IV) complex (about 16-fold higher).

A further test was performed to confirm the existence of ionic interactions between FMN and AGM-D by incubating 100 mg 1-FMN@AGM-D with 1 mL of PBS 1X and analyzing the resulting supernatant by UV-Vis. The amount of eluted FMN was determined by measuring its absorbance at 450 nm. As it can be concluded from FIG.2, almost all the FMN originally included in the catalytic system was lost after incubating it under buffer conditions. This indicates that the interaction was of ionic nature as being interrupted by the presence of the phosphate-buffered solution.

### 1.2. Catalytic conversion efficiency under light stimulus

In a first approach, the light conditions optimizing the substrate conversion were determined. To that end, 100 mg 1-FMN@AGM-D were incubated with 1 mL of 10 mM Tris-HCl at pH 7.6 for 5 minutes under irradiation with 460 nm low-power light (6 mW ·$cm^{-2}$). After such incubation, the suspension was vacuum filtered, the supernatant was kept (supernatant 1) and the solid (100 mg) was diluted again with 1 mL 1M NaCl for 10 minutes. After that second incubation, the suspension was vacuum filtered and the supernatant was kept (supernatant 2).

Once the experimental conditions were established, the following step was performed to confirm whether the catalytic system of the invention was able not only of fully converting the substrate in the desired drug, but also of allowing the complete delivery of the formed product from the matrix. To that end, supernatants 1 and 2 were analyzed by NMR and UPLC-MS as described above. As it is shown in FIG.3A, it was found that the 100% of substrate conversion was achieved when the low-power light was applied for only 5 minutes.

UPLC-MS confirmed that the photocatalytic reaction led to the generation and release of cisplatin in supernatant 1, yet without the need of adding NaCl to elute the drug (Fig.3B). Indeed, when light-irradiated 1-FMN@AGM-D were successively incubated with 1 M NaCl, UPLC-MS analysis showed no presence in the supernatant 2 of either Pt(IV) or cisplatin (Pt(II)) (Fig.3B). This result indicated that the compounds were no longer present in the polymeric matrix, consistently with the prodrug full conversion into cisplatin and its liberation from the beads before the salt washing. When kept in the dark, 1-FMN@AGM-D liberated approximately 80% of 1 after washing with 1 M NaCl (Fig.3B), yet no production of cisplatin was observed under these conditions, demonstrating that light is mandatory for the flavin-triggered catalytic activation of the prodrug.

Therefore, the catalytic system is not only remarkably efficient in the complete conversion of the starting Pt(IV)

prodrug concentration to Pt(II) but that the Pt(II) thus obtained is also successfully delivered to the medium.

On the one hand, this means that the light irradiation "activates" the catalyst and starts the reduction of Pt(IV) complex to Pt(II). The fact that the whole amount of prodrug can be successfully reduced means that the catalyst is able to "move" along and diffuse through the pores of the polymeric matrix by associating/dissociating electrostatic interactions and act over the electrostatically anchored Pt(IV) complexes. These electrostatic interactions are strong enough to maintain the catalyst within the porous matrix but weak enough to allow the access of the metal substrate to the catalyst.

On the other hand, it is also highlighted that the functionalization of the polymeric matrix is essential to allow the diffusion of the Pt(II). Once Pt(IV) is reduced, the metal complex loses its succinate ligands, which also disrupts the interaction with the positively-charged groups functionalizing the porous matrix. The loss of interaction eases the release of Pt(II) to the reaction medium. Conversely, succinate ligands have negative charges and need high salt concentrations to escape the AGM-D porous network.

1.3. Catalytic conversion efficiency in the presence of a co-factor

50 mg of the catalytic system obtained in previous section 1.1. (1-FMN@AGM-D) was incubated with 0.5 mL 1 mM NADH [GERBU] solution in 10 mM Tris-HCl at pH 7,6, for 15 minutes in dark. The accumulation of free succinate ligands along the time (at 5, 10 and 15 minutes) into the reaction bulk after addition of 1M NaCl was monitored by $^1$H NMR.

Monitoring the reaction progression, it was detected an increasing singlet peak at 2.4 ppm, which was indicative that succinate was released from the Pt(IV) complex as consequence of the NADH assisted flavin oxidation. The succinate ligand kept bound to the matrix until it was incubated with high salt concentration that elute the succinate molecules to the supernatant. Data demonstrated that NADH was able to reduce the immobilized FMN and concurrently transform the substrate Pt(IV) complex into cisplatin and free succinate in the dark.

In addition, it was found that the incubation of 1-FMN@AGM-D with NADH in the dark during 15 min reduced about 80% the immobilized Pt(IV) amount.-Also in the case of NADH-activated 1-FMN@AGM-D, EDX experiments showed disappearance of the Pt signal after light excitation (data not shown).

Altogether these data allow to conclude that the confinement of both FMN and NADH within the microporous environment of AGM-D triggers the reduction of Pt(IV) under dark conditions.

1.4. Catalytic conversion efficiency in the absence of $O_2$

In view of the importance of $O_2$ as electron acceptor in flavin catalysis and the relevance of hypoxic environments in cancer, the present inventors assessed the performance of light-irradiated 1-FMN@AGM-D under anoxic conditions.

For this purpose, the present inventors co-immobilized 10 mg of glucose oxidase (GOX) [Merck] on 1g of 1-FMN@AGM-D as obtained in previous section 1.1. to deplete $O_2$ in the catalytic system. The enzyme was mixed with 1-FMN@AGM-D for 30 minutes, then the suspension was vacuum filtered and the protein concentration of the supernatant was analyzed by Bradford method. 0.005 mL of supernatant were incubated with 0.2 mL of Braford reagent [MERCK] for 5 minutes in a plastic 96-well plate [Labbox]. The assay developed a blue color whose absorbance at 595 nm was measured by UV-Vis as described above. The protein concentration was calculated using a calibration curve of known concentrations of serum bovine albumin [Merck]. As result, quantitative immobilization of GOX was determined by measuring the protein concentration in the supernatant.

GOX selectively oxidizes glucose using $O_2$ as electron acceptor and producing gluconic acid and hydrogen peroxide. As an artificial anoxia is created within the bead microenvironment, the oxygen cannot now compete with Pt(IV)-complex as electron acceptor, enhancing the efficiency of 1-FMN-GOX@AGM-D in the photocatalytic reaction. 100 mg 1-FMN-GOX@AGM-D were incubated with 1 mL of 1M glucose and 10 mM Tris-HCl at pH 7,6 for 5 minutes under irradiation with 460 nm low-power light (6 mW $\cdot cm^{-2}$). After that incubation, the suspension was vacuum filtered, the supernatant was kept (supernatant 1) and the solid (100 mg) was diluted again with 1 mL 1M NaCl for 10 minutes. After that second incubation, the suspension was vacuum filtered and the supernatant was kept (supernatant 2). $^1$H NMR analysis of the supernatant revealed that the artificial anoxia triggered by GOX inside AGM-D prompted a 4-fold acceleration in the flavin-mediated cisplatin generation compared to the system without co-immobilized GOX described in 1.2 (aerobic conditions), upon the same light irradiation time. These studies were performed as described in the above methods.

Altogether these experimental data allow to conclude that GOX capability to reduce the availability of $O_2$ from glucose-rich settings, that is a very common scenario in tumor extracellular environments, can be of value in chemotherapy for indirectly speeding up the cisplatin photogeneration and delivery by 1-FMN@AGM-D.

1.5. Role of the functional groups in the matrix

As it has been shown above, the functional group DEAE anchors the Pt(IV) substrate efficiently and reversibly by electrostatic interactions within the polymeric matrix. The catalytic efficiency is not negatively affected by this anchoring, as

almost the 100% of the substrate has been converted into Pt(II) using a low-intensity light stimulus.

In this section, it was analyzed the role of the matrix in the REDOX reaction. To that end, the surface morphology of light-irradiated AGM-D by SEM (Scanning Electron Microscopy) [Carl Zeiss MERLIN™] upon loading of substrate and catalyst (as disclosed in section 1.1 above).

Fig.2 shows the AGM-D structure and porosity under different conditions. A and D for the agarose matrix alone, B and E for the catalytic system including both the Pt(IV) complex as well as the FMN catalyst at dark, and C and F for the catalytic system including both the Pt(IV) complex as well as the FMN catalyst once irradiated. As it is shown, loading of the Pt(IV) complex and FMN onto the beads kept in the dark, under the conditions pointed out in previous section 1.2., does not cause any significant change in the structure and porosity of AGM, while a notably rougher surface for 1-FMN@AGM-D after 5 min of low-light irradiation (460 nm, 6 mW $\cdot cm^{-2}$) was observed. Such change in morphology confirmed that the agarose microstructure, via its DEAE groups, should be participating in the catalytic cycle as electron donor.

Altogether this allows to conclude that the remarkable efficiency observed for 1-FMN@AGM-D is at least due to the fact that porous polymeric matrix AGM-D can directly supply electrons for the catalysis (i.e. via the agarose skeleton) besides functioning as chassis for the reaction.

Example 2. Preparation of AGM functionalized with boronic acid (AGM-B).

- Preparation of AGM

The AGM were functionalized with boronic acid groups (AGM-B) by firstly modifying AGM with epoxy groups using epichlorohydrine [Merck] in basic media (pH 11) during 16 hours at 25°C under vigorous stirring using a paddle stirrer at 100 rpm. After the epoxidation step, the AGM was washed with an excess of distilled water and finally incubated with a solution of 100 mM of 3-amino phenyl boronic acid in 100 mMM sodium bicarbonate buffer at pH 11 with 20% dimethyl formamide in a ratio 1:10 (w:v), during 16 hours at 25°C.

The resulting AGM-B was washed with an excess of water and the functionalized polymeric matrix was vacuum filtered and stored at 4°C. The confirmation that the resulting AGM was functionalized with boronic groups was made by X-ray photoelectron spectroscopy (XPS), confirming the presence of boronic groups (191.9 eV).

- Loading of FMN on AGM-B.

1 mL of a 1 mM solution of FMN (from Merck Ref: F2253) were incubated with 100 mg of AGM-B for 6 h at 25° C, under the ionic strength and pH conditions provided by 50 mM of phosphate buffer and 1 M NaCl (pH 8). The suspension was gently stirred with a wheel shaker (40 rpm).

It was also confirmed by UV-Vis, as it has been explained above, that the loading of the flavin into AGM-B was 0.8 $\mu$mol·g-1, by determining the intensity of the representative peak for FMN at 450 nm and converting it to concertation values according to the Beer-Lambert law.

The loading of the flavin catalyst into AGM-B was confirmed by UV-Vis (representative peak for FMN at 450 nm) as previously described for AGM-D. Loading of FMN was 0.8 $\mu$mol·g-1.

Example 3. Preparation of poly(methyl methacrylate) functionalized with positively-charged diethylaminoethyl groups (PMMA-D).

The microbeads of PMMA functionalized with epoxy groups [Purolite ECR8204F] are commercially available with a particle size range of 150-300 $\mu$m with a pores size range of 30-60 nm measured by SEM and according to the manufacture's instructions.

The PMMA was washed with an excess of distilled water and finally incubated with a solution of 1M of 2,2'-diethyl aminoethanol (DEAE) in a ratio 1:10 (w:v), during 16 hours at 25°C.

The resulting PMMA-D was washed with an excess of water and the functionalized polymeric matrix was vacuum filtered and stored at 4°C. The confirmation that the resulting PMMA was functionalized with DEAE was made by X-ray photoelectron spectroscopy (XPS), confirming the presence of -N bonds forming the secondary (398 eV) and tertiary amines (402 eV) from DEAE.

- Loading of FMN and 1 on PMMA-D

1 mL of a 75 $\mu$M FMN solution (from Merck Ref: F2253) were incubated with 100 mg PMMA-D for 10 minutes at 25° C, under the low ionic strength and neutral pH conditions provide by 10 mM Tris-HCl (pH 7.6). The suspension was gently stirred with a wheel shaker (30 rpm).

Subsequently, 1 mL of a 1 mM aqueous solution of the complex cis,cis,trans-$[Pt(NH_3)_2(Cl_2)$

$(O_2CCH_2CH_2CO_2H_2)_2]$ (1) (prepared as disclosed by Reithofer M. and colleagues (Reithofer M. et al., 2006)) was incubated with 100 mg PMMA-D loaded with FMN, under the same immobilization conditions previously described (10 minutes at 25°C at pH 7.5 in 10 mM Tris-HCl buffer). The suspension was gently stirred with a wheel shaker (30 rpm).

The loading of the flavin catalyst and metal prodrug into PMMA-D was confirmed by UV-Vis (representative peak for FMN at 450 nm and for Pt(IV)-complex at 220 nm) as previously described for AGM-D. Loading of the two components was 0.45 and 6.4 μmol·g-1, respectively.

In this catalytic system, hereinafter 1-FMN@PMMA-D, the final concentration of each one of the components was: 20 μmol DEAE, 0.45 μmol of FMN and 6.4 μmol Pt(IV) complex, all of them per gram of PMMA matrix. This means that DEAE groups were at a molar excess with respect to the catalyst (200-fold higher) and with respect to the substrate, the Pt(IV) complex (about 16-fold higher).

The catalytic conversion efficiency of 1-FMN@PMMA-D under light stimulus was assessed, as disclosed in the above section 1.2. It was found that the 60% of substrate conversion was achieved when the low-power light was applied for only 10 minutes.

**Citation List**

Alonso-de Castro S. et al., "Biological activity of Pt(IV) prodrugs triggered by riboflavin-mediated biorthogonal photocatalysis", Sci. Rep., 2108, 8, 17198.

Crocker L. et al., "Enzyme-inspired flavin-polydopamine as a biocompatible nanoparticle photocatalyst", Nanoscale Horizons, 2019, 4 (6), 1318-1325.

Greg Hermanson, "Bioconjugate Techniques", Chapter 1, "Introduction to Bioconjugation", Academic Press, 3rd edition, pages 1-125.

Haihua X. et al., "New polymer-platinum (II) antitumor conjugates", J Control Release, 2011, 152 Suppl. 1:e103-4.

Johnson E. M. et al., "Diffusion and partitioning of proteins in charged agarose gels", Biophys. J., 1995, 68(4), 1561-1568.

Laurent T. C. "Determination of the structure of agarose gels by gel chromatography", Biochimica et Biophysica Acta (BBA) - General Subjects, 1967, 136, 2, 199-205.

Pluen A. et al., "Diffusion of Macromolecules in Agarose Gels: Comparison of Linear and Globular Configurations", Biophysical Journal, 1999, 77, 542-552.

Pushkareva Z. V. et al. "Study of derivatives of the isoalloxazine series", Chemistry of Heterocyclic Compounds, 1966, volume 1, 406-408.

Reithofer M. et al., "An Entry to Novel Platinum Complexes: Carboxylation of Dihydroxoplatinum(IV) Complexes with Succinic Anhydride and Subsequent Derivatization", European Journal of Inorganic Chemistry, 2006, (13), 2612-2617.

Shen W. et al., "Thermogelling Polymer-Platinum(IV) Conjugates for Long-Term Delivery of Cisplatin", Biomacromolecules, 2015, 16, 105-115.

Wen S. et al., "An Amphiphilic Ruthenium Polymetallodrug for Combined Photodynamic Therapy and Photochemotherapy In Vivo", Advanced Materials, 2016, 29, 1603702.

Yoneda F. et al. "Syntheses of isoalloxazines and isoalloxazine 5-oxides. A new synthesis of riboflavin", J. Am. Chem. Soc., 1976, 98, 3, 830-835.

Wolinsky J. B. et al., "Local drug delivery strategies for cancer treatment: gels, nanoparticles, polymeric films, rods, and wafers", J Control Release, 2012, 159, 1, 14-26.

ALONSO-DE CASTRO SILVIA ET AL: "Bioorthogonal Catalytic Activation of Platinum and Ruthenium Anticancer

Complexes by FAD and Flavoproteins", ANGEWANDTE CHEMIE INTERNATIONAL EDITION, vol. 57, no. 12, 12 March 2018 (2018-03-12), pages 3143-3147

BENÍTEZ-MATEOS ET AL: "Selective Immobilization of Fluorescent Proteins for the Fabrication of Photoactive Materials", MOLECULES, vol. 24, no. 15, 1 August 2019 (2019-08-01), page 2775

## Claims

**1.** A catalytic system comprising a REDOX catalyst and a porous polymeric matrix wherein

- the porous polymeric matrix has pores with a size from 1 to 1000 nm and it is covalently functionalized with functional groups $R_1$ or, alternatively, with functional groups $R'_1$ and $R_2$,
- the REDOX catalyst comprises an isoalloxazine moiety which has one or more functional groups $R_3$,
- $R_1$ and $R_2$ are capable of forming non-covalent reversible interactions, selected from the group consisting of: electrostatic, hydrophobic, and hydrogen bonds, with $R_3$;
- $R_1$ and $R_2$ are in a molar concentration excess with respect to the molar concentration of the REDOX catalyst;
- $R_1$ and $R'_1$ are electron donor or electron acceptor groups having a REDOX potential between +2.70 to -2.5 V as determined by cyclic voltammetry using an Ag/AgCl electrode as reference.

**2.** The catalytic system of claim1, wherein the functional groups $R_3$ form an electrostatic interaction with the one or more functional groups $R_1$ or $R_2$ of the porous polymeric matrix, particularly with the $R_1$ functional group of the porous polymeric matrix.

**3.** The catalytic system of any one of the claims 1-2, wherein the porous polymeric matrix is covalently functionalized with $R_1$ charged functional groups which are selected from the group consisting of-$R_4N^{(+)}R_5R_6$, -$P^{(+)}R_7R_8R_9R_{10}$, -COO-, -$SO_{3-}$, -SO-, -$B(OH)_{2-}$, -$R_{11}$-$COO_-$, -$R_{12}$-$SO_{3-}$, -$R_{13}$-SO-, and -$R_{14}$-$B(OH)_{2-}$; wherein

$R_4$ to $R_{10}$ are the same or different and are independently selected from the group consisting of

· -H;
· ($C_1$-$C_{20}$) alkyl optionally substituted with one or more groups selected from $NR_{15}R_{16}$, nitro, hydroxyl, halogen, -O-$R_{35}$, ($C_1$-$C_3$)haloalkyl, ($C_3$-$C_7$) cycloalkyl, and aryl;
· ($C_2$-$C_{20}$) alkenyl optionally substituted with one or more groups selected from $NR_{17}R_{18}$, nitro, hydroxyl, halogen, -O-$R_{36}$, ($C_1$-$C_3$)haloalkyl, ($C_3$-$C_7$) cycloalkyl and aryl;
· ($C_1$-$C_{20}$) alkynyl optionally substituted with one or more groups selected from $NR_{19}R_{20}$, nitro, hydroxyl, halogen, -O-$R_{37}$, ($C_1$-$C_3$)haloalkyl, ($C_3$-$C_7$) cycloalkyl and aryl;
· ($C_3$-$C_7$) cycloalkyl optionally substituted with one or more groups selected from $NR_{21}R_{22}$, nitro, hydroxyl, halogen, -O-$R_{38}$, ($C_1$-$C_3$)haloalkyl, ($C_2$-$C_8$)alkenyl and ($C_2$-$C_8$)alkynyl; and
· ($C_5$-$C_6$) aromatic ring optionally substituted with one or more groups selected from $NR_{23}R_{24}$, nitro, hydroxyl, halogen, -O-$R_{39}$, ($C_1$-$C_3$)haloalkyl, ($C_2$-$C_8$)alkenyl and ($C_2$-$C_8$)alkynyl; or, alternatively, $R_4$, $R_5$ and $R_6$ form with the N atom a ($C_5$-$C_6$) saturated, partially saturated or aromatic ring system;

$R_{11}$ a $R_{14}$ are selected from the group consisting of

· ($C_1$-$C_{20}$) alkyl optionally substituted with one or more groups selected from $NR_{25}R_{26}$, nitro, hydroxyl, halogen, -O-$R_{39}$, ($C_1$-$C_8$)alkyl, ($C_1$-$C_8$)haloalkyl, ($C_2$-$C_8$)alkenyl, ($C_2$-$C_8$)haloalkenyl, ($C_3$-$C_7$) cycloalkyl, and aryl;
· ($C_2$-$C_{20}$) alkenyl optionally substituted with one or more groups selected from $NR_{27}R_{28}$, nitro, hydroxyl, halogen, -O-$R_{40}$, ($C_1$-$C_8$)alkyl, ($C_1$-$C_8$)haloalkyl, ($C_2$-$C_8$)alkenyl, ($C_2$-$C_8$)haloalkenyl, ($C_3$-$C_7$) cycloalkyl, and aryl;
· ($C_2$-$C_{20}$) alkynyl optionally substituted with one or more groups selected from $NR_{29}R_{30}$, nitro, hydroxyl, halogen, -O-$R_{41}$, ($C_1$-$C_8$)alkyl, ($C_1$-$C_8$)haloalkyl, ($C_3$-$C_7$) cycloalkyl, and aryl;
· ($C_3$-$C_7$) cycloalkyl optionally substituted with one or more groups selected from $NR_{31}R_{32}$, nitro, hydroxyl, halogen, -O-$R_{42}$, ($C_1$-$C_8$)alkyl, ($C_1$-$C_8$)haloalkyl, ($C_2$-$C_8$)alkenyl, ($C_2$-$C_8$)haloalkenyl, ($C_2$-$C_8$)alkynyl, ($C_2$-$C_8$)haloalkynyl; and
· ($C_5$-$C_6$) aromatic ring optionally substituted with one or more groups selected from $NR_{32}R_{34}$, nitro, hydroxyl, halogen, -O-$R_{43}$, ($C_1$-$C_8$)alkyl, ($C_1$-$C_8$)haloalkyl, ($C_2$-$C_8$)alkenyl, ($C_2$-$C_8$)haloalkenyl, ($C_2$-$C_8$)alkynyl,

$(C_2\text{-}C_8)$haloalkynyl;

$R_{15}$ to $R_{34}$ are the same or different and are selected from the group consisting of -H, $(C_1\text{-}C_{10})$alkyl, $(C_1\text{-}C_{10})$haloalkyl, $(C_2\text{-}C_{10})$alkenyl, $(C_2\text{-}C_{10})$alkynyl, $(C_3\text{-}C_7)$ cycloalkyl, and aryl; and
$R_{35}$ to $R_{43}$ are selected from $(C_1\text{-}C_{10})$alkyl, $(C_1\text{-}C_{10})$haloalkyl, $(C_2\text{-}C_{10})$alkenyl, $(C_2\text{-}C_{10})$alkynyl, $(C_3\text{-}C_7)$ cycloalkyl, and aryl.

**4.** The catalytic system of any one of the claims 1-3, wherein $R_1$ functional groups are positively charged functional groups and $R_3$ functional groups are negatively charged functional groups.

**5.** The catalytic system of claim 4, wherein "$R_1$" represents $-R_4N^{(+)}R_5R_6$ being $R_4$ to $R_6$ as defined in claim 3, particularly $(C_1\text{-}C_{20})$alkyl, $(C_2\text{-}C_{20})$alkenyl or $(C_2\text{-}C_{20})$alkynyl, particularly $(C_1\text{-}C_{20})$alkyl.

**6.** The catalytic system of any one of the preceding claims, wherein $R_3$ is a functional group selected from the group consisting of phosphate, sulphate, -COO-, $-SO_{3-}$, -SO-, $-B(OH)_{2-}$ and combinations thereof.

**7.** The catalytic system of any one of the preceding claims, wherein the REDOX catalyst is selected from the group consisting of riboflavin, FMN, FAD, flavoproteins and combinations thereof, particularly FMN.

**8.** The catalytic system of any one of the preceding claims which further comprises a substrate which is capable of being reduced or oxidized by the REDOX catalyst, the substrate comprising a reduceable or oxidizable moiety selected from the group consisting of: a C-N bond, a C-O bond, a C-S bond, a C-C bond, a C=N bond, a C=O bond, a C=S bond, a C=N bond, a C=C bond, a C≡C bond, a metal,a metal salt and combinations thereof.

**9.** The catalytic system of any one of the preceding claims, which further comprises at least one additional REDOX cofactor selected from the group consisting of: NADH/NAD+, glutathione disulfide/glutathione, pyruvate/lactate, dehydroascorbic acid/ascorbic acid, cytochrome C, Fe-S clusters, pyridoxal phosphate and combinations thereof.

**10.** The catalytic system of any one of the preceding claims, which is selected from the group consisting of:

Catalytic system I comprising (a) a porous polymeric matrix, particularly a cross-linked porous polymeric matrix, made of saccharide monomers, such as agarose, the matrix being functionalized with positively charged $R_1$ groups, and (b) an isoalloxazine-based REDOX catalyst with negatively charged $R_3$ groups, wherein the porous polymeric matrix has one or more of the following features: (i) it further includes a substrate, which is a metal-based substrate such as a metal salt or complex, which is electrostatically immobilized on the porous polymeric matrix, (ii) the $R_1$ functional groups are at a molar concentration excess with respect to the molar concentration of the catalyst, particularly the molar concentration ratio of $R_1$ functional groups vs molar concentration of catalyst is of at least 50, of at least 100, of at least 150, particularly of 200; (iii) the substrate, if present, is at a molar concentration excess with respect to the catalyst, and at a molar concentration defect with respect to $R_1$ functional groups; and (iv) the $R_1$ are electron donor groups, such as $-R_4N^{(+)}R_5R_6$, being $R_4$ to $R_6$ as defined above;
Catalytic system II comprising (a) a porous polymeric matrix, particularly a cross-linked porous polymeric matrix, made of saccharide monomers, such as agarose, the porous polymeric matrix having a porosity degree of at least 90% and a porous size comprised from 20 to 800 nm, from 40 to 600 nm, from 50 to 400 nm, from 60 to 300 nm or from 70 to 200 nm, the matrix being functionalized with positively charged $R_1$ groups, and (b) an isoalloxazine-based catalyst with negatively charged $R_3$ groups, wherein the porous polymeric matrix has one or more of the following features: (i) it further includes a substrate, which is a metal-based substrate such as a metal salt or complex, which is electrostatically immobilized on the porous polymeric matrix, (ii) the $R_1$ functional groups are at a molar concentration excess with respect to the molar concentration of catalyst, particularly the molar concentration ratio of $R_1$ functional groups vs molar concentration of catalyst is of at least 50, of at least 100, of at least 150, particularly of 200; (iii) the substrate, if present, is at a molar concentration excess with respect to the catalyst, and at a molar concentration defect with respect to $R_1$ functional groups; and (iv) the $R_1$ are electron donor groups, such as $--R_4N^{(+)}R_5R_6$, being $R_4$ to $R_6$ as defined above;
Catalytic system III comprising (a) a porous polymeric matrix, particularly a cross-linked porous polymeric matrix, made of saccharide monomers, such as agarose, the matrix being functionalized with positively charged $R_1$ groups, (b) an isoalloxazine-based catalyst with negatively charged $R_3$ groups, and (c) a substrate, which is a metal-based substrate such as a metal salt or complex, particular a metal prodrug, which is electrostatically immobilized to the porous polymeric matrix, wherein (i) the $R_1$ functional groups are at a molar concentration excess with respect to the catalyst, particularly the molar concentration ratio of $R_1$ functional groups vs molar

concentration of catalyst is of at least 50, of at least 100, of at least 150, particularly of 200; (ii) the substrate is at a molar concentration excess with respect to the catalyst and in a molar concentration defect with respect to $R_1$ functional groups, and (iii) the $R_1$ are electron donor groups, such as $-R_4N^{(+)}R_5R_6$, being $R_4$ to $R_6$ as defined above, particularly being the same, particularly being a $(C_1$-$C_{20})$alkyl;

Catalytic system IV comprising (a) a porous polymeric matrix, particularly a cross-linked porous polymeric matrix, made of saccharide monomers, such as agarose, the porous polymeric matrix having a porosity degree of at least 90% and a porous size from 20 to 800 nm, from 40 to 600 nm, from 50 to 400 nm, from 60 to 300 nm or from 70 to 200 nm, the matrix being functionalized with positively charged $R_1$ groups, (b) an isoalloxazine-based catalyst with negatively charged $R_3$ groups, and (c) a substrate, which is a metal-based substrate such as a metal salt or complex, particular a metal prodrug, which is electrostatically immobilized to porous polymeric matrix, wherein (i) the $R_1$ functional groups are at a molar concentration excess with respect to the catalyst, particularly the molar concentration ratio of $R_1$ functional groups vs molar concentration of catalyst is of at least 50, of at least 100, of at least 150, particularly of 200; (ii) the substrate is at a molar concentration excess with respect to the catalyst, and in a molar concentration defect with respect to $R_1$ functional groups, and (iii) the $R_1$ are electron donor groups, such as $-R_4N^{(+)}R_5R_6$, being $R_4$ to $R_6$ as defined above, particularly being the same, particularly being a $(C_1$-$C_{20})$ alkyl;

Catalytic system V comprising (a) a porous polymeric matrix, particularly a cross-linked porous polymeric matrix, made of saccharide monomers, such as agarose, the matrix being functionalized with positively charged $R_1$ groups, (b) an isoalloxazine-based catalyst, particularly FMN, with negatively charged $R_3$ groups, and (c) a substrate, which is a Pt(IV) complex prodrug which is electrostatically immobilized on the porous polymeric matrix (by interactions with the $R_1$ functional groups), wherein (i) the $R_1$ functional groups are at a molar concentration excess with respect to the catalyst, particularly the molar concentration ratio of $R_1$ functional groups vs molar concentration of catalyst is of at least 50, of at least 100, of at least 150, particularly of 200; (ii) the substrate is at a molar concentration excess with respect to the catalyst, and in a molar concentration defect with respect to $R_1$ functional groups, and (iii) the $R_1$ are electron donor groups, such as $-R_4N^{(+)}R_5R_6$, being $R_4$ to $R_6$ as defined above, particularly being the same, particularly being a $(C_1$-$C_{20})$alkyl; and

Catalytic system VI comprising (a) a porous polymeric matrix, particularly a cross-linked porous polymeric matrix, made of saccharide monomers, such as agarose, the porous polymeric matrix having a porosity degree of at least 90% and a porous size from 20 to 800 nm, from 40 to 600 nm, from 50 to 400 nm, from 60 to 300 nm or from 70 to 200 nm, the matrix being functionalized with positively charged $R_1$ groups, (b) an isoalloxazine-based catalyst, particularly FMN, with negatively charged $R_3$ groups, and (c) a substrate, which is a Pt(IV) complex prodrug which is electrostatically immobilized to porous polymeric matrix (by interactions with the $R_3$ functional groups, i.e., the phosphate groups in the case of FMN), wherein (i) the $R_1$ functional groups are at a molar concentration excess with respect to the catalyst, particularly the molar concentration ratio of $R_1$ functional groups vs molar concentration of catalyst is of at least 50, of at least 100, of at least 150, particularly of 200; (ii) the substrate is at a molar concentration excess with respect to the catalyst, and in a molar concentration defect with respect $R_1$ functional groups, and (iii) the $R_1$ are electron donor groups, such as $-R_4N^{(+)}R_5R_6$, being $R_4$ to $R_6$ as defined above, particularly being the same, particularly being a $(C_1$-$C_{20})$alkyl.

**11.** A process for preparing a catalytic system as defined in any one of the claims 1-10, the process comprising the step of mixing the porous polymeric matrix and the REDOX catalyst as defined in any of the preceding claims, wherein the catalyst is added at a molar concentration in defect with respect to the molar concentration of $R_1$ or $R_2$ functional groups, under appropriate conditions.

**12.** The process of claim 11, which further comprises the step of adding the substrate as defined in claim 8 at a molar concentration in defect with respect to the molar concentration of $R_1$ or $R_2$ and/or a REDOX co-factor.

**13.** A device comprising the catalytic system as defined in any one of the claims 1-10, such as a medical device or an industrial reactor.

**14.** A kit of parts comprising:

i) a compartment "A" comprising a porous polymeric matrix; a compartment "B" comprising the catalyst; and, optionally, a compartment "C" comprising a substrate, wherein the matrix, catalyst and substrate are as defined in any of the preceding claims 1-10; or, alternatively,

ii) a compartment "D" comprising the catalytic system as defined in any of the preceding claims 1-10, and, optionally, a compartment "E" comprising a substrate.

**15.** A catalytic system as defined in any one of the claims 1-10 for use in therapy or diagnostics.

**16.** A catalytic system as defined in any one of the claims 1-10 for use in the treatment or prevention of cancer, particularly for use in a method for treating or preventing cancer, the method comprising the steps of administering the catalytic device and applying an external stimuli selected from light, temperature, a REDOX cofactor and a combination thereof.

**17.** An ex vivo method for performing a REDOX catalytic reaction of a substrate comprising the steps of (a) contacting the substrate with the catalytic system as defined in any one of the claims 1-10; and (b) applying an external stimuli selected from light, temperature, a REDOX cofactor, differential voltage applied between electrodes, and a combination thereof.

**Patentansprüche**

**1.** Ein katalytisches System umfassend einen REDOX-Katalysator und eine poröse polymere Matrix, wobei

- die poröse polymere Matrix Poren mit einer Größe von 1 bis 1000 nm hat und mit funktionellen Gruppen $R_1$ oder ersatzweise mit funktionellen Gruppen $R'_1$ und $R_2$ kovalent funktionalisiert ist,
- der REDOX-Katalysator einen Isoalloxazinteil umfasst, der eine oder mehrere funktionelle Gruppen $R_3$ hat,
- $R_1$ und $R_2$ in der Lage sind, mit $R_3$ nicht-kovalente reversible Wechselwirkungen zu bilden, die aus der Gruppe ausgewählt sind, die aus den Folgenden besteht: elektrostatischen, hydrophoben und Wasserstoffbrückenbindungen;
- $R_1$ und $R_2$ in einem molaren Konzentrationsüberschuss in Bezug auf die molare Konzentration des REDOX-Katalysators vorliegen;
- $R_1$ und $R'_1$ Elektronendonator- oder Elektronenakzeptorgruppen mit einem REDOX-Potential zwischen +2,70 bis -2,5 V sind, wie durch zyklische Voltammetrie unter Verwendung einer Ag/AgCl-Elektrode als Referenz bestimmt.

**2.** Das katalytische System von Anspruch 1, wobei die funktionellen Gruppen $R_3$ eine elektrostatische Wechselwirkung mit der einen oder den mehreren funktionellen Gruppen $R_1$ oder $R_2$ der porösen polymeren Matrix bilden, insbesondere mit der funktionellen Gruppe $R_1$ der porösen polymeren Matrix.

**3.** Das katalytische System von einem der Ansprüche 1 bis 2, wobei die poröse polymere Matrix kovalent mit geladenen funktionellen Gruppen $R_1$ funktionalisiert ist, die ausgewählt sind aus der Gruppe bestehend aus- $R_4N^{(+)}R_5R_6$, $-P^{(+)}R_7R_8R_9R_{10}$, -COO-, $-SO_3$-, -SO-, $-B(OH)_2$-, $-R_{11}$-COO-, $-R_{12}-SO_3$-, $-R_{13}$-SO- und $-R_{14}-B(OH)_2$-; wobei

$R_4$ bis $R_{10}$ gleich oder verschieden sind und unabhängig voneinander ausgewählt sind aus der Gruppe bestehend aus

· -H;
· $(C_1-C_{20})$-Alkyl, wahlweise substituiert mit einer oder mehreren Gruppen, die ausgewählt sind aus $NR_{15}R_{16}$, Nitro, Hydroxy, Halogen, $-O-R_{35}$, $(C_1-C_8)$-Halogenalkyl, $(C_3-C_7)$-Cycloalkyl und Aryl;
· $(C_2-C_{20})$-Alkenyl, wahlweise substituiert mit einer oder mehreren Gruppen, die ausgewählt sind aus $NR_{17}R_{18}$, Nitro, Hydroxy, Halogen, $-O-R_{36}$, $(C_1-C_8)$-Halogenalkyl, $(C_3-C_7)$-Cycloalkyl und Aryl;
· $(C_1-C_{20})$-Alkinyl, wahlweise substituiert mit einer oder mehreren Gruppen, die ausgewählt sind aus $NR_{19}R_{20}$, Nitro, Hydroxy, Halogen, $-O-R_{37}$, $(C_1-C_8)$-Halogenalkyl, $(C_3-C_7)$-Cycloalkyl und Aryl;
· $(C_3-C_7)$-Cycloalkyl, wahlweise substituiert mit einer oder mehreren Gruppen, die ausgewählt sind aus $NR_{21}R_{22}$, Nitro, Hydroxy, Halogen, $-O-R_{38}$, $(C_1-C_8)$-Halogenalkyl, $(C_2-C_8)$-Alkenyl und $(C_2-C_8)$Alkinyl; und
· $(C_5-C_6)$ aromatischem Ring, wahlweise substituiert mit einer oder mehreren Gruppen, die ausgewählt sind aus $NR_{23}R_{24}$, Nitro, Hydroxy, Halogen, $-O-R_{39}$, $(C_1-C_8)$-Halogenalkyl, $(C_2-C_8)$-Alkenyl und $(C_2-C_8)$-Alkinyl; oder ersatzweise bilden $R_4$, $R_5$ und $R_6$ mit dem N-Atom ein $(C_5-C_6)$ gesättigtes, teilweise gesättigtes oder aromatisches Ringsystem;

$R_{11}$ a $R_{14}$ ausgewählt sind aus der Gruppe bestehend aus

· $(C_1-C_{20})$-Alkyl, wahlweise substituiert mit einer oder mehreren Gruppen, die ausgewählt sind aus $NR_{25}R_{26}$, Nitro, Hydroxy, Halogen, $-O-R_{39}$, $(C_1-C_8)$-Alkyl, $(C_1-C_8)$-Halogenalkyl, $(C_2-C_8)$-Alkenyl, $(C_2-C_8)$-Haloge-

nalkenyl, $(C_3-C_7)$-Cycloalkyl und Aryl;

· $(C_2-C_{20})$-Alkenyl, wahlweise substituiert mit einer oder mehreren Gruppen, die ausgewählt sind aus $NR_{27}R_{28}$, Nitro, Hydroxy, Halogen, $-O-R_{40}$, $(C_1-C_8)$-Alkyl, $(C_1-C_8)$-Halogenalkyl, $(C_2-C_8)$-Alkenyl, $(C_2-C_8)$-Halogenalkenyl, $(C_3-C_7)$-Cycloalkyl und Aryl;

· $(C_2-C_{20})$-Alkinyl, wahlweise substituiert mit einer oder mehreren Gruppen, die ausgewählt sind aus $NR_{29}R_{30}$, Nitro, Hydroxy, Halogen, $-O-R_{41}$, $(C_1-C_8)$-Alkyl, $(C_1-C_8)$-Halogenalkyl, $(C_3-C_7)$-Cycloalkyl und Aryl;

· $(C_3-C_7)$-Cycloalkyl, wahlweise substituiert mit einer oder mehreren Gruppen, die ausgewählt sind aus $NR_{31}R_{32}$, Nitro, Hydroxy, Halogen, $-O-R_{42}$, $(C_1-C_8)$-Alkyl, $(C_1-C_8)$-Halogenalkyl, $(C_2-C_8)$-Alkenyl, $(C_2-C_8)$-Halogenalkenyl, $(C_2-C_8)$-Alkinyl, $(C_2-C_8)$-Halogenalkinyl; und

· $(C_5-C_6)$ aromatischem Ring, wahlweise substituiert mit einer oder mehreren Gruppen, die ausgewählt sind aus $NR_{32}R_{34}$, Nitro, Hydroxy, Halogen, $-O-R_{43}$, $(C_1-C_8)$-Alkyl, $(C_1-C_8)$-Halogenalkyl, $(C_2-C_8)$-Alkenyl, $(C_2-C_8)$-Halogenalkenyl, $(C_2-C_8)$-Alkinyl, $(C_2-C_8)$Halogenalkinyl;

$R_{15}$ bis $R_{34}$ gleich oder verschieden sind und ausgewählt sind aus der Gruppe bestehend aus -H, $(C_1-C_{10})$-Alkyl, $(C_1-C_{10})$-Halogenalkyl, $(C_2-C_{10})$-Alkenyl, $(C_2-C_{10})$-Alkinyl, $(C_3-C_7)$-Cycloalkyl und Aryl; und
$R_{35}$ bis $R_{43}$ ausgewählt sind aus $(C_1-C_{10})$-Alkyl, $(C_1-C_{10})$-Halogenalkyl, $(C_2-C_{10})$-Alkenyl, $(C_2-C_{10})$-Alkinyl, $(C_3-C_7)$-Cycloalkyl und Aryl.

**4.** Das katalytische System von einem der Ansprüche 1 bis 3, wobei die funktionellen Gruppen $R_1$ positiv geladene funktionelle Gruppen sind und die funktionellen Gruppen $R_3$ negativ geladene funktionelle Gruppen sind.

**5.** Das katalytische System von Anspruch 4, wobei "$R_1$" $-R_4N^{(+)}R_5R_6$ bedeutet, wobei $R_4$ bis $R_6$ wie in Anspruch 3 definiert sind, insbesondere $(C_1-C_{20})$-Alkyl, $(C_2-C_{20})$-Alkenyl oder $(C_2-C_{20})$-Alkinyl, insbesondere $(C_1-C_{20})$-Alkyl.

**6.** Das katalytische System von einem der vorhergehenden Ansprüche, wobei $R_3$ eine funktionelle Gruppe ist, die ausgewählt ist aus der Gruppe bestehend aus Phosphat, Sulfat, -COO-, $-SO_3$-, -SO-, - $B(OH)_2$- und Kombinationen davon.

**7.** Das katalytische System von einem der vorhergehenden Ansprüche, wobei der REDOX-Katalysator ausgewählt ist aus der Gruppe bestehend aus Riboflavin, FMN, FAD, Flavoproteinen und Kombinationen davon, insbesondere FMN.

**8.** Das katalytische System von einem der vorhergehenden Ansprüche, das ferner ein Substrat umfasst, das durch den REDOX-Katalysator reduziert oder oxidiert werden kann, wobei das Substrat einen reduzierbaren oder oxidierbaren Teil umfasst, der ausgewählt ist aus der Gruppe bestehend aus: einer C-N-Bindung, einer C-O-Bindung, einer C-S-Bindung, einer C-C-Bindung, einer C=N-Bindung, einer C=O-Bindung, einer C=S-Bindung, einer C=N-Bindung, einer C= C-Bindung, einer C≡ C-Bindung, einem Metall, einem Metallsalz und Kombinationen davon.

**9.** Das katalytische System von einem der vorhergehenden Ansprüche, das ferner mindestens einen zusätzlichen REDOX-Cofaktor umfasst, der ausgewählt ist aus der Gruppe bestehend aus: NADH/NAD+, Glutathiondisulfid/Glutathion, Pyruvat/Lactat, Dehydroascorbinsäure/Ascorbinsäure, Cytochrom C, Fe-S-Clustern, Pyridoxalphosphat und Kombinationen davon.

**10.** Das katalytische System von einem der vorhergehenden Ansprüche, das ausgewählt ist aus der Gruppe bestehend aus:

dem katalytische System I umfassend (a) eine poröse polymere Matrix, insbesondere eine vernetzte poröse polymere Matrix, bestehend aus Saccharidmonomeren, wie z. B. Agarose, wobei die Matrix mit positiv geladenen $R_1$-Gruppen funktionalisiert ist, und (b) einen REDOX-Katalysator auf Isoalloxazinbasis mit negativ geladenen $R_3$-Gruppen, wobei die poröse polymere Matrix eines oder mehrere der folgenden Merkmale hat: (i) es beinhaltet ferner ein Substrat, das ein Substrat auf Metallbasis ist, wie z. B. ein Metallsalz oder -komplex, das elektrostatisch auf der porösen polymeren Matrix immobilisiert ist, (ii) die funktionellen Gruppen $R_1$ liegen in einem molaren Konzentrationsüberschuss in Bezug auf die molare Konzentration des Katalysators vor, insbesondere liegt das molare Konzentrationsverhältnis von den funktionellen Gruppen $R_1$ zur molaren Konzentration des Katalysators bei mindestens 50, bei mindestens 100, bei mindestens 150, insbesondere bei 200 vor; (iii) das Substrat liegt, falls vorhanden, in einem molaren Konzentrationsüberschuss in Bezug auf den Katalysator und in einem molaren Konzentrationsmangel in Bezug auf die funktionellen Gruppen $R_1$ vor; und (iv) die $R_1$ sind Elektronendonator-

gruppen, wie z. B. $-R_4N^{(+)}R_5R_6$, wobei $R_4$ bis $R_6$ wie oben definiert sind;

dem katalytischen System II umfassend (a) eine poröse polymere Matrix, insbesondere eine vernetzte poröse polymere Matrix, bestehend aus Saccharidmonomeren, wie z. B. Agarose, wobei die poröse polymere Matrix einen Porositätsgrad von mindestens 90 % und eine poröse Größe reichend von 20 bis 800 nm, von 40 bis 600 nm, von 50 bis 400 nm, von 60 bis 300 nm oder von 70 bis 200 nm hat, wobei die Matrix mit positiv geladenen $R_1$-Gruppen funktionalisiert ist, und (b) einen Katalysator auf Isoalloxazinbasis mit negativ geladenen $R_3$-Gruppen, wobei die poröse polymere Matrix eines oder mehrere der folgenden Merkmale hat: (i) es beinhaltet ferner ein Substrat, das ein Substrat auf Metallbasis ist, wie z. B. ein Metallsalz oder -komplex, das elektrostatisch auf der porösen polymeren Matrix immobilisiert ist, (ii) die funktionellen Gruppen $R_1$ liegen in einem molaren Konzentrationsüberschuss in Bezug auf die molare Konzentration des Katalysators vor, insbesondere liegt das molare Konzentrationsverhältnis von den funktionellen Gruppen $R_1$ zur molaren Konzentration des Katalysators bei mindestens 50, bei mindestens 100, bei mindestens 150, insbesondere bei 200 vor; (iii) das Substrat liegt, falls vorhanden, in einem molaren Konzentrationsüberschuss in Bezug auf den Katalysator und in einem molaren Konzentrationsmangel in Bezug auf die funktionellen Gruppen $R_1$ vor; und (iv) die $R_1$ sind Elektronendonatorgruppen, wie z. B. $-R_4N^{(+)}R_5R_6$, wobei $R_4$ bis $R_6$ wie oben definiert sind;

dem katalytischen System III, umfassend (a) eine poröse polymere Matrix, insbesondere eine vernetzte poröse polymere Matrix, bestehend aus Saccharidmonomeren wie z. B. Agarose, wobei die Matrix mit positiv geladenen $R_1$-Gruppen funktionalisiert ist, (b) einen Katalysator auf Isoalloxazinbasis mit negativ geladenen $R_3$-Gruppen und (c) ein Substrat, das ein Substrat auf Metallbasis, wie z. B. ein Metallsalz oder -komplex, insbesondere ein Metallprodrug ist, das elektrostatisch auf der porösen polymeren Matrix immobilisiert ist, wobei (i) die funktionellen $R_1$-Gruppen in einem molaren Konzentrationsüberschuss in Bezug auf den Katalysator vorliegen, insbesondere liegt das molare Konzentrationsverhältnis von den $R_1$ funktionellen Gruppen zu der molaren Konzentration des Katalysators bei mindestens 50, bei mindestens 100, bei mindestens 150, insbesondere bei 200 vor; (ii) das Substrat liegt in einem molaren Konzentrationsüberschuss in Bezug auf den Katalysator und in einem molaren Konzentrationsmangel in Bezug auf die funktionellen Gruppen $R_1$ vor, und (iii) die $R_1$ Elektronendonatorgruppen, wie z. B. $-R_4N^{(+)}R_5R_6$ sind, wobei $R_4$ bis $R_6$ wie oben definiert, insbesondere gleich, insbesondere ein $(C_1\text{-}C_{20})$-Alkyl sind;

dem katalytischen System IV, umfassend (a) eine poröse polymere Matrix, insbesondere eine vernetzte poröse polymere Matrix, bestehend aus Saccharidmonomeren, wie z. B. Agarose, wobei die poröse polymere Matrix einen Porositätsgrad von mindestens 90% und eine poröse Größe von 20 bis 800 nm, von 40 bis 600 nm, von 50 bis 400 nm, von 60 bis 300 nm oder von 70 bis 200 nm hat, wobei die Matrix mit positiv geladenen $R_1$-Gruppen funktionalisiert ist, (b) einen Katalysator auf Isoalloxazinbasis mit negativ geladenen $R_3$-Gruppen, und (c) ein Substrat, das ein metallbasiertes Substrat, wie z. B. ein Metallsalz oder -komplex, insbesondere ein Metallprodrug, ist, das elektrostatisch auf einer poröse polymere Matrix immobilisiert ist, wobei (i) die funktionellen Gruppen $R_1$ in einem molaren Konzentrationsüberschuss in Bezug auf den Katalysator vorliegen, insbesondere liegt das molare Konzentrationsverhältnis von den funktionellen Gruppen $R_1$ zu der molaren Konzentration des Katalysators bei mindestens 50, bei mindestens 100, bei mindestens 150, insbesondere bei 200 vor; (ii) das Substrat in einem molaren Konzentrationsüberschuss in Bezug auf den Katalysator und in einem molaren Konzentrationsmangel in Bezug auf die funktionellen Gruppen $R_1$ vorliegt, und (iii) die $R_1$ Elektronendonatorgruppen, wie z. B. - $R_4N^{(+)}R_5R_6$ sind, wobei $R_4$ bis $R_6$ wie oben definiert sind, insbesondere gleich sind, insbesondere ein $(C_1\text{-}C_{20})$-Alkyl sind;

dem katalytischen System V umfassend (a) eine poröse polymere Matrix, insbesondere eine vernetzte poröse polymere Matrix, bestehend aus Saccharidmonomeren, wie z. B. Agarose, wobei die Matrix mit positiv geladenen $R_1$-Gruppen funktionalisiert ist, (b) einen Katalysator auf Isoalloxazinbasis, insbesondere FMN, mit negativ geladenen $R_3$-Gruppen, und (c) ein Substrat, das ein Pt(IV)-Komplex-Prodrug ist, das auf der porösen polymeren Matrix (durch Wechselwirkungen mit den funktionellen Gruppen $R_1$) elektrostatisch immobilisiert ist, wobei (i) die funktionellen Gruppen $R_1$ in einem molaren Konzentrationsüberschuss in Bezug auf den Katalysator vorliegen, insbesondere liegt das molare Konzentrationsverhältnis von den funktionellen Gruppen $R_1$ zu der molaren Konzentration des Katalysators bei mindestens 50, bei mindestens 100, bei mindestens 150, insbesondere bei 200 vor; (ii) das Substrat in einem molaren Konzentrationsüberschuss in Bezug auf den Katalysator und in einem molaren Konzentrationsmangel in Bezug auf die funktionellen Gruppen $R_1$ vorliegt, und (iii) die $R_1$ Elektronendonatorgruppen, wie z. B. $-R_4N^{(+)}R_5R_6$ sind, wobei $R_4$ bis $R_6$ wie oben definiert sind, insbesondere gleich sind, insbesondere ein $(C_1\text{-}C_{20})$-Alkyl sind; und

dem katalytischen System VI, umfassend (a) eine poröse polymere Matrix, insbesondere eine vernetzte poröse polymere Matrix, aus Saccharidmonomeren, wie z. B. Agarose, wobei die poröse polymere Matrix einen Porositätsgrad von mindestens 90% und eine poröse Größe von 20 bis 800 nm, von 40 bis 600 nm, von 50 bis 400 nm, von 60 bis 300 nm oder von 70 bis 200 nm hat, wobei die Matrix mit positiv geladenen $R_1$-Gruppen funktionalisiert ist, (b) einen Katalysator auf Isoalloxazinbasis, insbesondere FMN, mit negativ geladenen

$R_3$-Gruppen, und (c) ein Substrat, das ein Pt(IV)-Komplex-Prodrug ist, das elektrostatisch auf der porösen polymeren Matrix (durch Wechselwirkungen mit den funktionellen Gruppen $R_3$, d. h. den Phosphatgruppen im Falle von FMN) immobilisiert ist, wobei (i) die funktionellen Gruppen $R_1$ in einem molaren Konzentrationsüberschuss in Bezug auf den Katalysator vorliegen, insbesondere liegt das molare Konzentrationsverhältnis von den funktionellen Gruppen $R_1$ zu der molaren Konzentration des Katalysators bei mindestens 50, bei mindestens 100, bei mindestens 150, insbesondere bei 200 vor; (ii) das Substrat in einem molaren Konzentrationsüberschuss in Bezug auf den Katalysator und in einem molaren Konzentrationsmangel in Bezug auf die funktionellen Gruppen $R_1$ vorliegt, und (iii) die $R_1$ Elektronendonatorgruppen, wie z. B. $-R_4N^{(+)}R_5R_6$ sind, wobei $R_4$ bis $R_6$ wie oben definiert sind, insbesondere die gleichen sind, insbesondere ein ($C_1$-$C_{20}$)-Alkyl sind.

**11.** Ein Verfahren zur Herstellung eines katalytischen Systems wie in einem der Ansprüche 1 bis 10 definiert, wobei das Verfahren den Schritt des Mischens der porösen polymeren Matrix und des REDOX-Katalysators wie in einem der vorhergehenden Ansprüche definiert umfasst, wobei der Katalysator in einer molaren Mangelkonzentration in Bezug auf die molare Konzentration der funktionellen Gruppen $R_1$ oder $R_2$ unter geeigneten Bedingungen zugegeben wird.

**12.** Das Verfahren von Anspruch 11, das ferner den Schritt des Zugebens des Substrats wie in Anspruch 8 definiert in einer molaren Mangelkonzentration in Bezug auf die molare Konzentration von $R_1$ oder $R_2$ und/oder einem REDOX-Cofaktor umfasst.

**13.** Eine Vorrichtung umfassend das katalytische System wie in einem der Ansprüche 1 bis 10 definiert, wie z. B. eine medizinische Vorrichtung oder einen industriellen Reaktor.

**14.** Ein Teilesatz, umfassend:

i) eine Abteilung "A", die eine poröse polymere Matrix umfasst; eine Abteilung "B", die den Katalysator umfasst; und wahlweise eine Abteilung "C", umfassend ein Substrat, wobei die Matrix, der Katalysator und das Substrat wie in einem der vorhergehenden Ansprüche 1 bis 10 definiert sind; oder, ersatzweise
ii) eine Abteilung "D", die das katalytische System wie in einem der vorhergehenden Ansprüche 1 bis 10 definiert umfasst, und wahlweise eine Abteilung "E", die ein Substrat umfasst.

**15.** Ein katalytisches System wie in einem der Ansprüche 1 bis 10 definiert zur Verwendung in der Therapie oder Diagnostik.

**16.** Ein katalytisches System wie in einem der Ansprüche 1 bis 10 definiert zur Verwendung bei der Behandlung oder Prävention von Krebs, insbesondere zur Verwendung in einem Verfahren zur Behandlung oder Prävention von Krebs, wobei das Verfahren die Schritte der Verabreichung der katalytischen Vorrichtung und der Anwendung eines externen Stimulus, ausgewählt aus Licht, Temperatur, einem REDOX-Cofaktor und einer Kombination davon, umfasst.

**17.** Ein ex-vivo-Verfahren zum Durchführen einer katalytischen REDOX-Reaktion eines Substrats, umfassend die folgenden Schritte: (a) in-Kontakt-bringen des Substrats mit dem katalytischen System wie in einem der Ansprüche 1 bis 10 definiert; und (b) Anwenden eines externen Stimulus, der ausgewählt ist aus Licht, Temperatur, einem REDOX-Cofaktor, einer zwischen Elektroden angelegten Differenzspannung und einer Kombination davon.

## Revendications

**1.** Un système catalytique comprenant un catalyseur REDOX et une matrice polymère poreuse dans lequel

- la matrice polymère poreuse a des pores ayant une taille de 1 à 1000 nm et elle est fonctionnalisée de manière covalente avec des groupes fonctionnels $R_1$ ou, en variante, avec des groupes fonctionnels $R'_1$ et $R_2$,
- le catalyseur REDOX comprend une fraction isoalloxazine qui a un ou plusieurs groupes fonctionnels $R_3$,
- $R_1$ et $R_2$ sont capables de former des interactions réversibles non covalentes, choisies dans le groupe constitué par : des liaisons électrostatiques, hydrophobes et d'hydrogène, avec $R_3$ ;
- $R_1$ et $R_2$ sont en excès de concentration molaire par rapport à la concentration molaire du catalyseur REDOX ;
- $R_1$ et $R'_1$ sont des groupes donneurs ou accepteurs d'électrons ayant un potentiel REDOX compris entre +2,70 et -2,5 V tel que déterminé par voltamétrie cyclique en utilisant une électrode Ag/AgCl comme référence.

2. Le système catalytique de la revendication 1, dans lequel les groupes fonctionnels $R_3$ forment une interaction électrostatique avec l'un ou les plusieurs groupes fonctionnels $R_1$ ou $R_2$ de la matrice polymère poreuse, en particulier avec le groupe fonctionnel $R_1$ de la matrice polymère poreuse.

3. Le système catalytique de l'une quelconque des revendications 1 à 2, dans lequel la matrice polymère poreuse est fonctionnalisée de manière covalente avec des groupes fonctionnels chargés $R_1$ qui sont choisis dans le groupe constitué par- $R_4N^{(+)}R_5R_6$, $-P^{(+)}R_7R\ _8R_9R_{10}$, -COO-, $-SO_3^-$, -SO-, $-B(OH)_2^-$, $-R_{11}-COO^-$, $-R_{12}-SO_3^-$, $-R_{13}-SO^-$ et $-R_{14}-B(OH)_2^-$; dans lequel

   $R_4$ à $R_{10}$ sont identiques ou différents et sont choisis indépendamment dans le groupe constitué par

   · -H;
   · alkyle en ($C_1$-$C_{20}$) éventuellement substitué par un ou plusieurs groupes choisis parmi $NR_{15}R_{16}$, nitro, hydroxyle, halogène, $-O-R_{35}$, halogénoalkyle en ($C_1$-$C_8$), cycloalkyle en ($C_3$-$C_7$) et aryle ;
   · alcényle en ($C_2$-$C_{20}$) éventuellement substitué par un ou plusieurs groupes choisis parmi $NR_{17}R_{18}$, nitro, hydroxyle, halogène, $-O-R_{36}$, halogénoalkyle en ($C_1$-$C_8$), cycloalkyle en ($C_3$-$C_7$) et aryle ;
   · alcynyle en ($C_1$-$C_{20}$) éventuellement substitué par un ou plusieurs groupes choisis parmi $NR_{19}R_{20}$, nitro, hydroxyle, halogène, $-O-R_{37}$, halogénoalkyle en ($C_1$-$C_8$), cycloalkyle en ($C_3$-$C_7$) et aryle ;
   · cycloalkyle en ($C_3$-$C_7$) éventuellement substitué par un ou plusieurs groupes choisis parmi $NR_{21}R_{22}$, nitro, hydroxyle, halogène, $-O-R_{38}$, halogénoalkyle en ($C_1$-$C_8$), alcényle en ($C_2$-$C_8$) et alcynyle en ($C_2$-$C_8$) ; et
   · cycle aromatique en ($C_5$-$C_6$) éventuellement substitué par un ou plusieurs groupes choisis parmi $NR_{23}R_{24}$, nitro, hydroxyle, halogène, $-O-R_{39}$, halogénoalkyle en ($C_1$-$C_8$), alcényle en ($C_2$-$C_8$) et alcynyle en ($C_2$-$C_8$) ;
   ou, en variante, $R_4$, $R_5$ et $R_6$ forment avec l'atome N un système cyclique saturé, partiellement saturé ou aromatique en ($C_5$-$C_6$) ;

   $R_{11}$ a $R_{14}$ sont choisis dans le groupe constitué par

   · alkyle en ($C_1$-$C_{20}$) éventuellement substitué par un ou plusieurs groupes choisis parmi $NR_{25}R_{26}$, nitro, hydroxyle, halogène, $-O-R_{39}$, alkyle en ($C_1$-$C_8$), halogénoalkyle en ($C_1$-$C_8$), alcényle en ($C_2$-$C_8$), halogénoalcényle en ($C_2$-$C_8$), cycloalkyle en ($C_3$-$C_7$) et aryle ;
   · alcényle en ($C_2$-$C_{20}$) éventuellement substitué par un ou plusieurs groupes choisis parmi $NR_{27}R_{28}$, nitro, hydroxyle, halogène, $-O-R_{40}$, alkyle en ($C_1$-$C_8$), halogénoalkyle en ($C_1$-$C_8$), alcényle en ($C_2$-$C_8$), halogénoalcényle en ($C_2$-$C_8$), cycloalkyle en ($C_3$-$C_7$) et aryle ;
   · alcynyle en ($C_2$-$C_{20}$) éventuellement substitué par un ou plusieurs groupes choisis parmi $NR_{29}R_{30}$, nitro, hydroxyle, halogène, $-O-R_{41}$, alkyle en ($C_1$-$C_8$), halogénoalkyle en ($C_1$-$C_8$), cycloalkyle en ($C_3$-$C_7$) et aryle ;
   · cycloalkyle en ($C_3$-$C_7$) éventuellement substitué par un ou plusieurs groupes choisis parmi $NR_{31}R_{32}$, nitro, hydroxyle, halogène, $-O-R_{42}$, alkyle en ($C_1$-$C_8$), halogénoalkyle en ($C_1$-$C_8$), alcényle en ($C_2$-$C_8$), halogénoalcényle en ($C_2$-$C_8$), alcynyle en ($C_2$-$C_8$), halogénoalcynyle en ($C_2$-$C_8$) ; et
   · cycle aromatique en ($C_5$-$C_6$) éventuellement substitué par un ou plusieurs groupes choisis parmi $NR_{32}R_{34}$, nitro, hydroxyle, halogène, $-O-R_{43}$, alkyle en ($C_1$-$C_8$), halogénoalkyle en ($C_1$-$C_8$), alcényle en ($C_2$-$C_8$), halogénoalcényle en ($C_2$-$C_8$), alcynyle en ($C_2$-$C_8$), halogénoalcynyle en ($C_2$-$C_8$) ;

   $R_{15}$ à $R_{34}$ sont identiques ou différents et sont choisis dans le groupe constitué par -H, alkyle en ($C_1$-$C_{10}$), halogénoalkyle en ($C_1$-$C_{10}$), alcényle en ($C_2$-$C_{10}$), alcynyle en ($C_2$-$C_{10}$), cycloalkyle en ($C_3$-$C_7$) et aryle ; et
   $R_{35}$ à $R_{43}$ sont choisis parmi alkyle en ($C_1$-$C_{10}$), halogénoalkyle en ($C_1$-$C_{10}$), alcényle en ($C_2$-$C_{10}$), alcynyle en ($C_2$-$C_{10}$), cycloalkyle en ($C_3$-$C_7$) et aryle.

4. Le système catalytique de l'une quelconque des revendications 1 à 3, dans lequel les groupes fonctionnels $R_1$ sont des groupes fonctionnels chargés positivement et les groupes fonctionnels $R_3$ sont des groupes fonctionnels chargés négativement.

5. Le système catalytique de la revendication 4, dans lequel « $R_1$ » représente $-R_4N^{(+)}R_5$, R $_6$ étant R $_4$ à $R_6$ tels que définis dans la revendication 3, en particulier un alkyle en ($C_1$-$C_{20}$), un alcényle en ($C_2$-$C_{20}$) ou un alcynyle en ($C_2$-$C_{20}$), en particulier un alkyle en ($C_1$-$C_{20}$).

6. Le système catalytique de l'une quelconque des revendications précédentes, dans lequel $R_3$ est un groupe fonctionnel choisi dans le groupe constitué par le phosphate, le sulfate, -COO-, $-SO_3^-$, - $SO^-$, $-B(OH)_2^-$ et des combinaisons de ceux-ci.

7. Le système catalytique de l'une quelconque des revendications précédentes, dans lequel le catalyseur REDOX est choisi dans le groupe constitué par la riboflavine, la FMN, la FAD, les flavoprotéines et des combinaisons de celles-ci, en particulier la FMN.

8. Le système catalytique de l'une quelconque des revendications précédentes qui comprend en outre un substrat qui est capable d'être réduit ou oxydé par le catalyseur REDOX, le substrat comprenant une fraction réductible ou oxydable choisie dans le groupe constitué par : une liaison C-N, une liaison C-O, une liaison C-S, une liaison C-C, une liaison C=N, une liaison C=O, une liaison C=S, une liaison C=N, une liaison C=C, une liaison C=C, un métal, un sel de métal et des combinaisons de ceux-ci.

9. Le système catalytique de l'une quelconque des revendications précédentes, qui comprend en outre au moins un cofacteur REDOX additionnel choisi dans le groupe constitué par : NADH/NAD+, disulfure de glutathion/glutathion, pyruvate/lactate, acide déhydroascorbique/acide ascorbique, cytochrome C, agrégats de Fe-S, phosphate de pyridoxal et des combinaisons de ceux-ci.

10. Le système catalytique de l'une quelconque des revendications précédentes, qui est choisi dans le groupe constitué par :

le système catalytique I comprenant (a) une matrice polymère poreuse, en particulier une matrice polymère poreuse réticulée, constituée de monomères de saccharide, tels que l'agarose, la matrice étant fonctionnalisée avec des groupes $R_1$ chargés positivement, et (b) un catalyseur REDOX à base d'isoalloxazine avec des groupes $R_3$ chargés négativement, dans lequel la matrice polymère poreuse a une ou plusieurs des caractéristiques suivantes : (i) elle comprend en outre un substrat, qui est un substrat à base de métal tel qu'un sel ou un complexe de métal, qui est immobilisé électrostatiquement sur la matrice polymère poreuse, (ii) les groupes fonctionnels $R_1$ sont en excès de concentration molaire par rapport à la concentration molaire du catalyseur, en particulier le rapport de concentration molaire des groupes fonctionnels $R_1$ par rapport à la concentration molaire du catalyseur est d'au moins 50, d'au moins 100, d'au moins 150, en particulier de 200 ; (iii) le substrat, s'il est présent, est en excès de concentration molaire par rapport au catalyseur, et en défaut de concentration molaire par rapport aux groupes fonctionnels $R_1$ ; et (iv) les $R_1$ sont des groupes donneurs d'électrons, tels que $-R_4N^{(+)}R_5R_6$, étant $R_4$ à $R_6$ tels que définis ci-dessus ;

le système catalytique II comprenant (a) une matrice polymère poreuse, en particulier une matrice polymère poreuse réticulée, constituée de monomères de saccharide, tels que l'agarose, la matrice polymère poreuse ayant un degré de porosité d'au moins 90 % et une taille poreuse comprise de 20 à 800 nm, de 40 à 600 nm, de 50 à 400 nm, de 60 à 300 nm ou de 70 à 200 nm, la matrice étant fonctionnalisée avec des groupes $R_1$ chargés positivement, et (b) un catalyseur à base d'isoalloxazine avec des groupes $R_3$ chargés négativement, dans lequel la matrice polymère poreuse a une ou plusieurs des caractéristiques suivantes : (i) elle comprend en outre un substrat, qui est un substrat à base de métal tel qu'un sel ou un complexe de métal, qui est immobilisé électrostatiquement sur la matrice polymère poreuse, (ii) les groupes fonctionnels $R_1$ sont en excès de concentration molaire par rapport à la concentration molaire du catalyseur, en particulier le rapport de concentration molaire des groupes fonctionnels $R_1$ par rapport à la concentration molaire du catalyseur est d'au moins 50, d'au moins 100, d'au moins 150, en particulier de 200 ; (iii) le substrat, s'il est présent, est en excès de concentration molaire par rapport au catalyseur, et en défaut de concentration molaire par rapport aux groupes fonctionnels $R_1$ ; et (iv) les $R_1$ sont des groupes donneurs d'électrons, tels que $-R_4N^{(+)}R_5R_6$, étant $R_4$ à $R_6$ tels que définis ci-dessus ;

le système catalytique III comprenant (a) une matrice polymère poreuse, en particulier une matrice polymère poreuse réticulée, constituée de monomères de saccharide, tels que l'agarose, la matrice étant fonctionnalisée avec des groupes $R_1$ chargés positivement, (b) un catalyseur à base d'isoalloxazine avec des groupes $R_3$ chargés négativement, et (c) un substrat, qui est un substrat à base de métal tel qu'un sel ou un complexe de métal, en particulier un promédicament de métal, qui est immobilisé électrostatiquement sur la matrice polymère poreuse, dans lequel (i) les groupes fonctionnels $R_1$ sont en excès de concentration molaire par rapport au catalyseur, en particulier le rapport de concentration molaire des groupes fonctionnels $R_1$ par rapport à la concentration molaire du catalyseur est d'au moins 50, d'au moins 100, d'au moins 150, en particulier de 200 ; (ii) le substrat est en excès de concentration molaire par rapport au catalyseur et en défaut de concentration molaire par rapport aux groupes fonctionnels $R_1$, et (iii) les $R_1$ sont des groupes donneurs d'électrons, tels que $-R_4N^{(+)}R_5R_6$, étant $R_4$ à $R_6$ tels que définis ci-dessus, en particulier étant les mêmes, en particulier étant un alkyle en $(C_1-C_{20})$ ;

le système catalytique IV comprenant (a) une matrice polymère poreuse, en particulier une matrice polymère poreuse réticulée, constituée de monomères de saccharide, tels que l'agarose, la matrice polymère poreuse

ayant un degré de porosité d'au moins 90 % et une taille poreuse de 20 à 800 nm, de 40 à 600 nm, de 50 à 400 nm, de 60 à 300 nm ou de 70 à 200 nm, la matrice étant fonctionnalisée avec des groupes $R_1$ chargés positivement, (b) un catalyseur à base d'isoalloxazine avec des groupes $R_3$ chargés négativement, et (c) un substrat, qui est un substrat à base de métal tel qu'un sel ou un complexe de métal, en particulier un promédicament de métal, qui est immobilisé électrostatiquement sur une matrice polymère poreuse, dans lequel (i) les groupes fonctionnels $R_1$ sont en excès de concentration molaire par rapport au catalyseur, en particulier le rapport de concentration molaire des groupes fonctionnels $R_1$ par rapport à la concentration molaire du catalyseur est d'au moins 50, d'au moins 100, d'au moins 150, en particulier de 200 ; (ii) le substrat est en excès de concentration molaire par rapport au catalyseur, et dans un défaut de concentration molaire par rapport aux groupes fonctionnels $R_1$, et (iii) les $R_1$ sont des groupes donneurs d'électrons, tels que $-R_4N^{(+)}R_5R_6$, étant $R_4$ à $R_6$ tels que définis ci-dessus, étant en particulier les mêmes, étant en particulier un alkyle en ($C_1$-$C_{20}$) ;
le système catalytique V comprenant (a) une matrice polymère poreuse, en particulier une matrice polymère poreuse réticulée, constituée de monomères de saccharide, tels que l'agarose, la matrice étant fonctionnalisée avec des groupes $R_1$ chargés positivement, (b) un catalyseur à base d'isoalloxazine, en particulier la FMN, avec des groupes $R_3$ chargés négativement, et (c) un substrat, qui est un promédicament complexe de Pt(IV) qui est immobilisé électrostatiquement sur la matrice polymère poreuse (par des interactions avec les groupes fonctionnels $R_1$), dans lequel (i) les groupes fonctionnels $R_1$ sont en excès de concentration molaire par rapport au catalyseur, en particulier le rapport de concentration molaire des groupes fonctionnels $R_1$ par rapport à la concentration molaire du catalyseur est d'au moins 50, d'au moins 100, d'au moins 150, en particulier de 200 ; (ii) le substrat est en excès de concentration molaire par rapport au catalyseur et en défaut de concentration molaire par rapport aux groupes fonctionnels $R_1$, et (iii) les $R_1$ sont des groupes donneurs d'électrons, tels que $-R_4N^{(+)}R_5R_6$, étant $R_4$ à $R_6$ tels que définis ci-dessus, étant en particulier les mêmes, étant en particulier un alkyle en ($C_1$-$C_{20}$) ; et
le système catalytique VI comprenant (a) une matrice polymère poreuse, en particulier une matrice polymère poreuse réticulée, constituée de monomères de saccharide, tels que l'agarose, la matrice polymère poreuse ayant un degré de porosité d'au moins 90 % et une taille poreuse de 20 à 800 nm, de 40 à 600 nm, de 50 à 400 nm, de 60 à 300 nm ou de 70 à 200 nm, la matrice étant fonctionnalisée avec des groupes $R_1$ chargés positivement, (b) un catalyseur à base d'isoalloxazine, en particulier la FMN, avec des groupes $R_3$ chargés négativement, et (c) un substrat, qui est un promédicament complexe de Pt(IV) qui est immobilisé électrostatiquement sur une matrice polymère poreuse (par des interactions avec les groupes fonctionnels $R_3$, c'est-à-dire les groupes phosphate dans le cas de la FMN), dans lequel (i) les groupes fonctionnels $R_1$ sont en excès de concentration molaire par rapport au catalyseur, en particulier le rapport de concentration molaire des groupes fonctionnels $R_1$ par rapport à la concentration molaire du catalyseur est d'au moins 50, d'au moins 100, d'au moins 150, en particulier de 200 ; (ii) le substrat est en excès de concentration molaire par rapport au catalyseur, et en défaut de concentration molaire par rapport aux groupes fonctionnels $R_1$, et (iii) les $R_1$ sont des groupes donneurs d'électrons, tels que $-R_4N^{(+)}R_5R_6$, étant $R_4$ à $R_6$ tels que définis ci-dessus, en particulier étant les mêmes, en particulier étant un alkyle en ($C_1$-$C_{20}$).

**11.** Un procédé de préparation d'un système catalytique tel que défini dans l'une quelconque des revendications 1 à 10, le procédé comprenant l'étape consistant à mélanger la matrice polymère poreuse et le catalyseur REDOX tel que défini dans l'une quelconque des revendications précédentes, dans lequel le catalyseur est ajouté à une concentration molaire en défaut par rapport à la concentration molaire de groupes fonctionnels $R_1$ ou $R_2$, dans des conditions appropriées.

**12.** Le procédé de la revendication 11, qui comprend en outre l'étape consistant à ajouter le substrat tel que défini dans la revendication 8 à une concentration molaire en défaut par rapport à la concentration molaire de $R_1$ ou $R_2$ et/ou un cofacteur REDOX.

**13.** Un dispositif comprenant le système catalytique tel que défini dans l'une quelconque des revendications 1 à 10, tel qu'un dispositif médical ou un réacteur industriel.

**14.** Un kit de pièces comprenant :

i) un compartiment « A » comprenant une matrice polymère poreuse ; un compartiment « B » comprenant le catalyseur ; et, éventuellement, un compartiment « C » comprenant un substrat, dans lequel la matrice, le catalyseur et le substrat sont tels que définis dans l'une quelconque des revendications 1 à 10 précédentes ; ou, en variante,
ii) un compartiment « D » comprenant le système catalytique tel que défini dans l'une quelconque des

revendications 1 à 10 précédentes, et, éventuellement, un compartiment « E » comprenant un substrat.

**15.** Un système catalytique tel que défini dans l'une quelconque des revendications 1 à 10 pour son utilisation en thérapie ou en diagnostic.

**16.** Un système catalytique tel que défini dans l'une quelconque des revendications 1 à 10 pour son utilisation dans le traitement ou la prévention du cancer, en particulier pour son utilisation dans un procédé de traitement ou de prévention du cancer, le procédé comprenant les étapes d'administration du dispositif catalytique et d'application d'un stimulus externe choisi parmi la lumière, la température, un cofacteur REDOX et une combinaison de ceux-ci.

**17.** Un procédé ex vivo pour effectuer une réaction catalytique REDOX d'un substrat comprenant les étapes consistant à (a) mettre en contact le substrat avec le système catalytique tel que défini dans l'une quelconque des revendications 1 à 10 ; et (b) appliquer un stimulus externe choisi parmi la lumière, la température, un cofacteur REDOX, une tension différentielle appliquée entre les électrodes, et une combinaison de ceux-ci.

FIG. 1

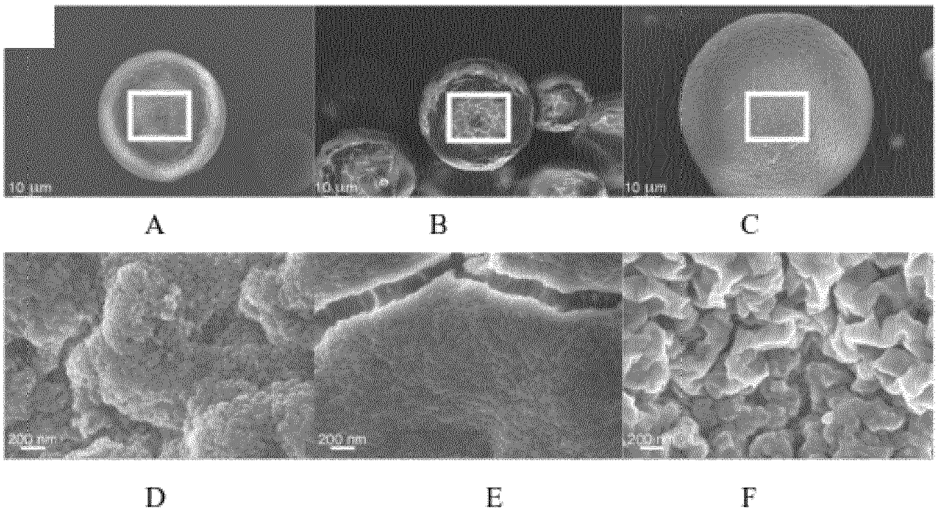
10 µm
10 µm
10 µm
A
B
C
200 nm
200 nm
200 nm
D
E
F

FIG. 2

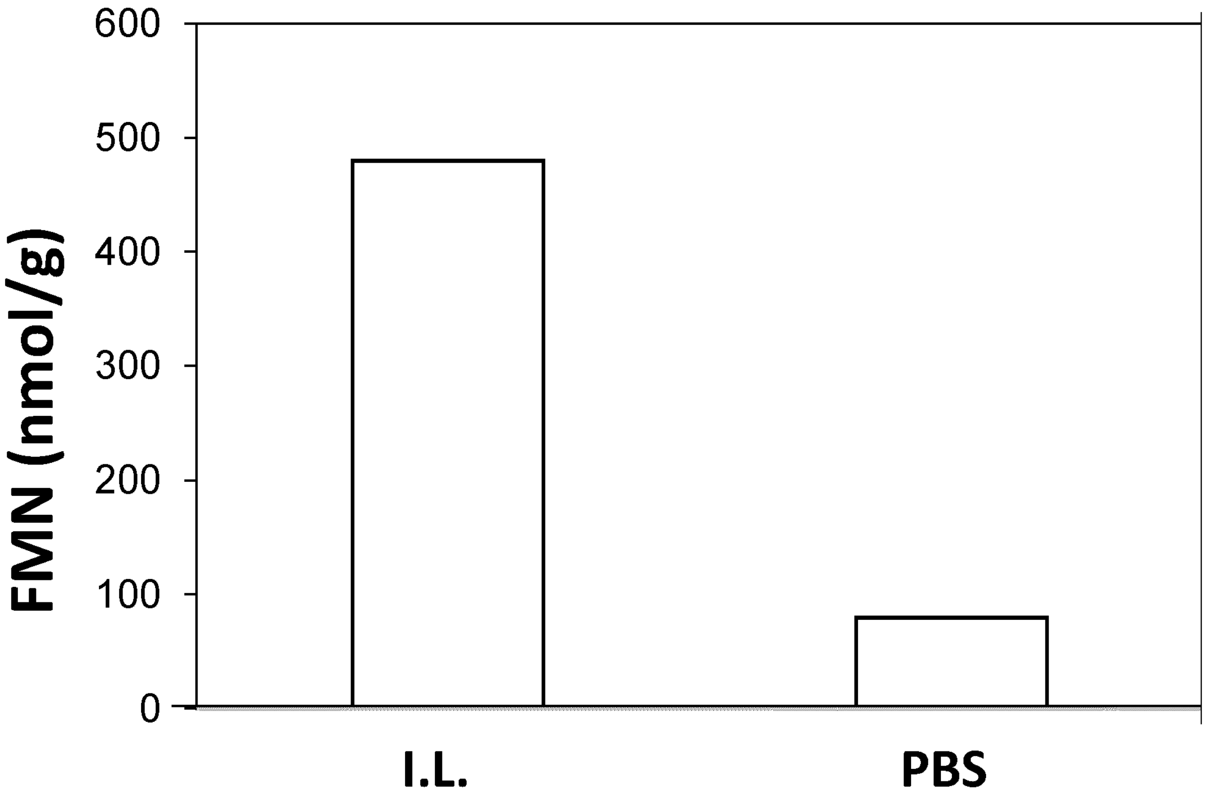
600
500
400
300
200
100
0
FMN (nmol/g)
I.L.
PBS

FIG. 3

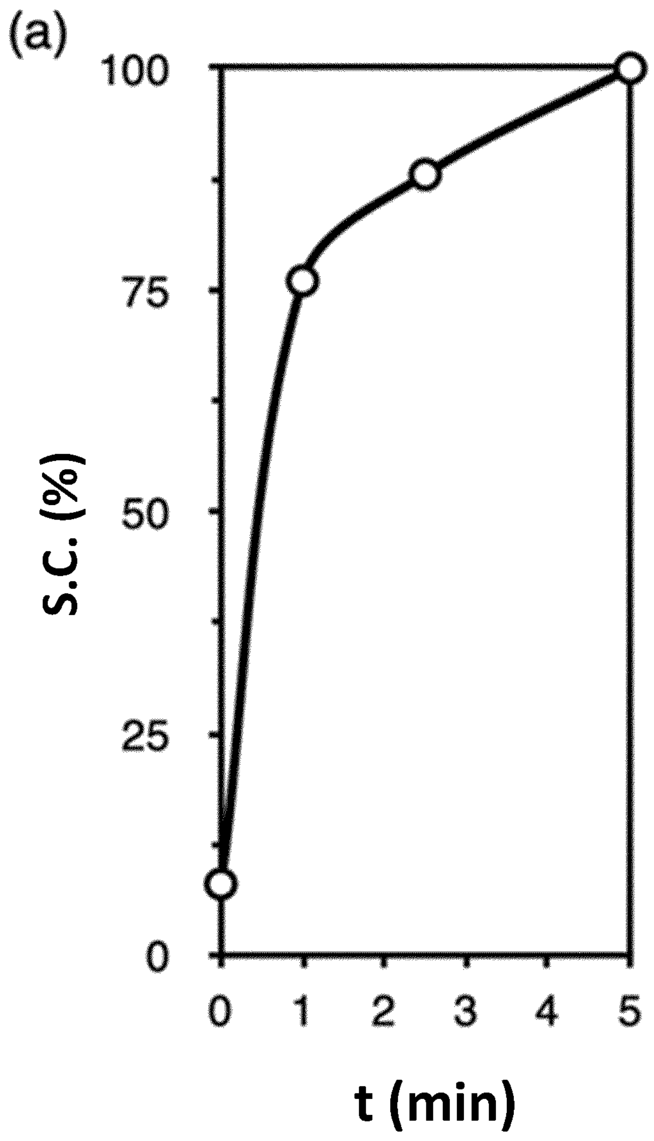

FIG. 3 (continuation)

b)

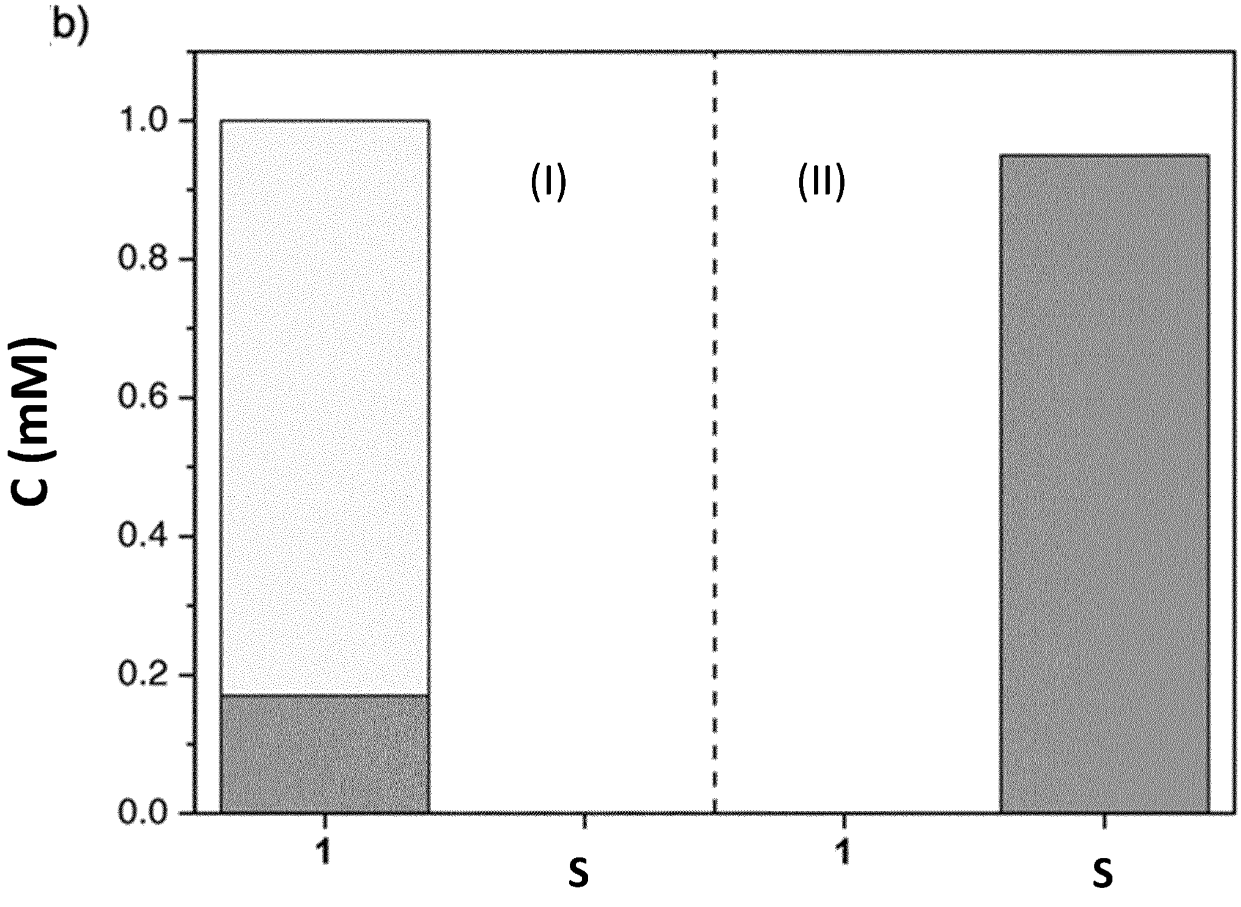

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Introduction to Bioconjugation. **GREG HERMANSON**. Bioconjugate Techniques. Academic Press, 1-125
- **ALONSO-DE CASTRO S. et al.** Biological activity of Pt(IV) prodrugs triggered by riboflavin-mediated biorthogonal photocatalysis. *Sci. Rep.*, vol. 8, 17198
- **CROCKER L. et al.** Enzyme-inspired flavin-polydopamine as a biocompatible nanoparticle photocatalyst. *Nanoscale Horizons*, 2019, vol. 4 (6), 1318-1325
- **HAIHUA X. et al.** New polymer-platinum (II) antitumor conjugates. *J Control Release*, 2011, vol. 152 (1), e103-4
- **JOHNSON E. M. et al.** Diffusion and partitioning of proteins in charged agarose gels. *Biophys. J.*, 1995, vol. 68 (4), 1561-1568
- **LAURENT T. C.** Determination of the structure of agarose gels by gel chromatography. *Biochimica et Biophysica Acta (BBA) - General Subjects*, 1967, vol. 136 (2), 199-205
- **PLUEN A. et al.** Diffusion of Macromolecules in Agarose Gels: Comparison of Linear and Globular Configurations. *Biophysical Journal*, 1999, vol. 77, 542-552
- **PUSHKAREVA Z. V. et al.** Study of derivatives of the isoalloxazine series. *Chemistry of Heterocyclic Compounds*, 1966, vol. 1, 406-408
- **REITHOFER M. et al.** An Entry to Novel Platinum Complexes: Carboxylation of Dihydroxoplatinum(IV) Complexes with Succinic Anhydride and Subsequent Derivatization. *European Journal of Inorganic Chemistry*, 2006, vol. 13, 2612-2617
- **SHEN W. et al.** Thermogelling Polymer-Platinum(IV) Conjugates for Long-Term Delivery of Cisplatin. *Biomacromolecules*, 2015, vol. 16, 105-115
- **WEN S. et al.** An Amphiphilic Ruthenium Polymetallodrug for Combined Photodynamic Therapy and Photochemotherapy In Vivo. *Advanced Materials*, 2016, vol. 29, 1603702
- **YONEDA F. et al.** Syntheses of isoalloxazines and isoalloxazine 5-oxides. A new synthesis of riboflavin. *J. Am. Chem. Soc.*, 1976, vol. 98 (3), 830-835
- **WOLINSKY J. B. et al.** Local drug delivery strategies for cancer treatment: gels, nanoparticles, polymeric films, rods, and wafers. *J Control Release*, 2012, vol. 159 (1), 14-26
- **ALONSO-DE CASTRO SILVIA et al.** Bioorthogonal Catalytic Activation of Platinum and Ruthenium Anticancer Complexes by FAD and Flavoproteins. *ANGEWANDTE CHEMIE INTERNATIONAL EDITION*, 12 March 2018, vol. 57 (12), 3143-3147
- **BENÍTEZ-MATEOS et al.** Selective Immobilization of Fluorescent Proteins for the Fabrication of Photoactive Materials. *MOLECULES*, 01 August 2019, vol. 24 (15), 2775